(19) Europäisches Patentamt
European Patent Office
Office européen des brevets

(11) EP 3 971 157 A1

(12) # EUROPEAN PATENT APPLICATION
published in accordance with Art. 153(4) EPC

(43) Date of publication:
**23.03.2022 Bulletin 2022/12**

(21) Application number: **20857681.9**

(22) Date of filing: **30.07.2020**

(51) International Patent Classification (IPC):
***C07C 7/12*** *(2006.01)* ***C07C 11/04*** *(2006.01)*
***B01D 53/047*** *(2006.01)* ***B01J 20/22*** *(2006.01)*
***B01J 20/34*** *(2006.01)*

(52) Cooperative Patent Classification (CPC):
**B01D 53/047; B01J 20/22; B01J 20/34; C07C 7/12; C07C 11/04**

(86) International application number:
**PCT/JP2020/029335**

(87) International publication number:
**WO 2021/039273 (04.03.2021 Gazette 2021/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **23.08.2019 JP 2019152875**

(71) Applicant: **Showa Denko K.K.**
**Tokyo 105-8518 (JP)**

(72) Inventors:
- **WATANABE, Yoshihiro**
  **Oita-shi, Oita 870-0189 (JP)**
- **OKUMURA, Yoshikuni**
  **Oita-shi, Oita 870-0189 (JP)**

(74) Representative: **Strehl Schübel-Hopf & Partner**
**Maximilianstrasse 54**
**80538 München (DE)**

(54) **METHOD FOR RECOVERING OLEFIN**

(57) Provided is a method for recovering, by pressure swing adsorption, unreacted olefins from a stream of a chemical reaction process in which an olefin is used as a material, the method enables desorption of gas at a relatively high desorption operation pressure, more preferably at a pressure not lower than the atmospheric pressure, and enables reuse of a separation agent. As the separation agent, a metal complex is used, in which pressure P3 at which a local maximum of dA/dP is obtained during adsorption and pressure P4 at which a local maximum of dA/dP is obtained during desorption are located between an adsorption operation pressure P1 and a desorption operation pressure P2, where dA/dP represents a value obtained by differentiating A by P, assuming that an olefin adsorption amount (A) is a function of an adsorption pressure (P), i.e., A = f(P), on an adsorption isotherm indicating the pressure (P) and the adsorption amount (A).

FIG. 2

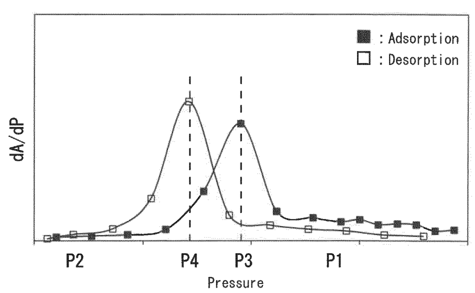


EP 3 971 157 A1

## Description

FIELD

The present disclosure relates to a method for recovering an unreacted olefin from a chemical reaction process using the olefin as a raw material. More particularly, the present disclosure relates to a method for recovering an olefin using a reusable metal complex as a separation agent.

BACKGROUND

In chemical reaction processes using an olefin as a raw material, such as a process in which ethylene or propylene is reacted with oxygen gas and acetic acid to produce vinyl acetate or allyl acetate; a process in which ethylene or propylene is polymerized to produce polyethylene or polypropylene; a process in which ethyl acetate is produced from ethylene and acetic acid; and a process in which acetic acid is produced from ethylene and oxygen gas, an unreacted raw material olefin is often reused as a circulating gas. This reuse is explained by using a schematic diagram of a chemical reaction process using an olefin as a raw material shown in FIG. 3. A raw material olefin is sent to a reactor, and separated into a liquid containing a large amount of a target substance and a gas containing a large amount of an unreacted raw material olefin by a separator. The liquid containing a large amount of a target substance is temporarily stored in a crude tank, and then sent to a purification system. The gas containing a large amount of an unreacted olefin is mixed with a raw material added thereto, and sent to the reactor as a circulating gas. This circulating gas contains impurities originally contained in a trace amount in the raw material olefin, nitrogen gas for pressure compensation, etc., and a part of the circulating gas is extracted from the system as an exhaust gas and discarded, in order to suppress these impurities and nitrogen gas from accumulating in the process. Since this extracted exhaust gas contains a large amount of the raw material olefin, it is desirable to recover the olefin.

Porous metal complexes useful for separating a specific gas from a mixed gas have been developed. For example, Patent Literature 1 and Patent Literature 2 describe porous metal complexes which selectively adsorb only a specific gas while accompanied by a change in the structure or size of pores. Further, Patent Literature 2 and Non-Patent Literature 1 also describe flexible porous metal complexes, the structures of which change depending on the type of gas to be contacted therewith, and methods for separating a gas using the same. In addition, Patent Literature 3 describes a porous metal complex, in which characteristics of a gate type polymer complex which exhibits gas adsorption in a gate manner and an I type complex which adsorbs a gas in a type I manner are changed by a switching material. The types of adsorption isotherm are described in Non-Patent Literature 2. Non-Patent Literature 1 describes a metal complex exhibiting an adsorption isotherm of a gate-open type in which an adsorbed amount rapidly increases at or above a predetermined pressure although gas is hardly adsorbed up to the predetermined pressure. Note that "porous" as used herein means that a metal complex has a special structure (void) at a molecular level, with a size capable of accommodating or releasing a hydrocarbon molecule to be adsorbed. In the case of a metal complex having a flexible structure, the shape and/or size of the void may change due to a structural change caused by an external stimulus, such as pressure.

These documents describe, as a method for desorbing an adsorbed gas from a porous metal complex when used in an actual industrial separation process, a method of reducing pressure using a vacuum pump or the like, a method of heating, and a method of heating and vacuuming.

[CITATION LIST]

[PATENT LITERATURE]

[PTL 1] JP 2009-208028 A
[PTL 2] JP 4994398 B
[PTL 3] JP 4834048 B

[NON-PATENT LITERATURE]

[NPL 1] Kazuhiro UEMURA, and Susumu KITAGAWA, Expected materials for the future, vol. 2, pp. 44-51 (2002)
[NPL 2] Yasushi TAKEUCHI, "Adsorption Separation", Baifukan, p. 35 (2000)

SUMMARY

[TECHNICAL PROBLEM]

When such a material which adsorbs a gas is used as a separation agent in a process of an actual industrial pressure swing adsorption method, it is necessary that the adsorbed gas can be desorbed from the separation agent and reused. However, many separation agents exhibit a type I adsorption isotherm as described in Non-Patent Literature 2, and in general, cannot be sufficiently desorb a gas at normal pressure, and therefore, a desorption operation pressure P2 should be lower than normal pressure, and the pressure should be reduced by using a vacuum pump, etc. The desorption of a gas by blowdown is not a preferable method since it requires a capital investment and an energy cost of operation.

It is an object of the present invention to provide a method for recovering an olefin, in which an unreacted olefin is recovered from a stream of a chemical reaction process using the olefin as a raw material by a pressure swing adsorption method, wherein a gas can be desorbed at a relatively high desorption operation pressure, more preferably at a pressure equal to or higher than normal pressure, and a separation agent can be reused.

[SOLUTION TO PROBLEM]

As a result of intensive studies to solve the aforementioned problems, the present inventors have found that, on the basis of an adsorption isotherm indicating an adsorption pressure of a separation agent (adsorbent) (P) and an olefin adsorbed amount (A), by using as a separation agent a metal complex in which a pressure P3 corresponding to a local maximum value at the time of adsorption and a pressure P4 corresponding to a local maximum value at the time of desorption exist between an adsorption operation pressure PI and a desorption operation pressure P2, with respect to a value (dA/dP) obtained by differentiating A with respect to P, in the case that the adsorbed amount (A) is expressed as a function of the adsorption pressure (P), A = f(P), the olefin can be easily desorbed and recovered at the pressure P2, thereby completing the present invention. That is, the present invention encompasses the following [1] to [8].

[1] A method for recovering an olefin, in which an unreacted olefin is recovered from a stream of a chemical reaction process using the olefin as a raw material by a pressure swing adsorption method, wherein, on the basis of an adsorption isotherm indicating an adsorption pressure (P) and an olefin adsorbed amount (A), a metal complex in which a pressure P3 corresponding to a local maximum value at the time of adsorption and a pressure P4 corresponding to a local maximum value at the time of desorption exist between an adsorption operation pressure PI and a desorption operation pressure P2, with respect to a value (dA/dP) obtained by differentiating A with respect to P, in the case that the adsorbed amount (A) is expressed as a function of the adsorption pressure (P), A = f(P), is used as a separation agent.

[2] The method for recovering an olefin according to [1], wherein, in an adsorption isotherm during an adsorption operation of the metal complex, an olefin adsorbed amount A1 at the adsorption operation pressure PI and an olefin adsorbed amount A2 at the desorption operation pressure P2 satisfy a relationship of $(A1 - A2) \times P2 / ((PI - P2) \times A2) \geq 0.4$.

[3] The method for recovering an olefin according to [1] or [2], wherein the desorption operation pressure P2 is equal to or higher than normal pressure.

[4] The method for recovering an olefin according to any one of [1] to [3], wherein the number of carbon atoms of the olefin is 2 to 4.

[5] The method for recovering an olefin according to any one of [1] to [4], wherein the olefin is ethylene.

[6] The method for recovering an olefin according to any one of [1] to [5], wherein the chemical reaction process is any one of a vinyl acetate production process, an ethyl acetate production process, an allyl acetate production process, a polyethylene production process, and a polypropylene production process.

[7] The method for recovering an olefin according to any one of [1] to [6], wherein the metal complex is composed of a metal ion and an organic ligand, and the organic ligand is at least one organic compound selected from the group consisting of (1) to (3) below.

(1) An organic compound capable of bidentate coordination to the metal ion, and having two or more carboxy groups and/or hydroxy groups in the molecule, and having no heterocycle

(2) An organic compound capable of bidentate coordination to the metal ion, and having a monocyclic or polycyclic, saturated or unsaturated heterocycle having one heteroatom selected from N, O or S, and a carboxy group or a hydroxy group in the molecule

(3) An organic compound capable of bidentate coordination to the metal ion, and having a monocyclic or polycyclic, saturated or unsaturated heterocycle having two or more heteroatoms selected from the group consisting of N, O and S in the molecule

[8] The method for recovering an olefin according to any one of [1] to [7], wherein the metal complex is composed of a metal ion and an organic ligand, and the organic ligand is one or more organic compounds selected from the group consisting of an alkylene dicarboxylic acid compound having 4 to 20 carbon atoms; an alkenylene dicarboxylic acid compound having 4 to 20 carbon atoms; a dicarboxylic acid compound represented by the following general formulas (I) to (III):

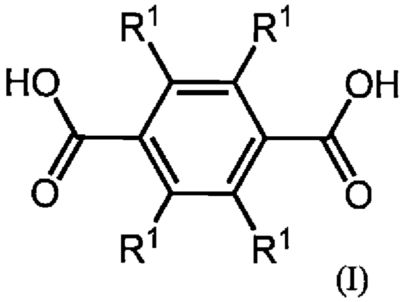

(I)

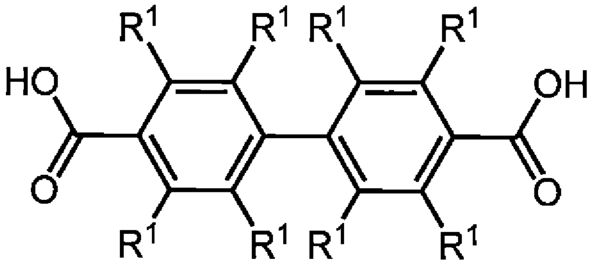

(II)

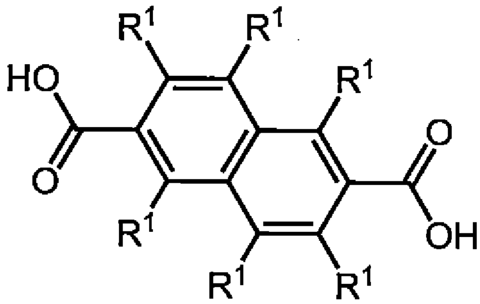

(III)

wherein in the formulas, $R^1$s are each independently a hydrogen atom, a halogen atom, an alkyl group having 1 to 4 carbon atoms, an alkoxy group having 1 to 4 carbon atoms, a formyl group, an acyloxy group having 1 to 4 carbon atoms, an alkoxycarbonyl group having an alkoxy group having 1 to 4 carbon atoms, a nitro group, a cyano group, a carboxy group, an amino group, a monoalkylamino group having 1 to 4 carbon atoms, a dialkylamino group having alkyl groups having 1 to 4 carbon atoms, or an acylamino group having 1 to 4 carbon atoms, and two or more of $R^1$s may be condensed to form a ring; a dicarboxylic acid compound represented by the following general formula (IV):

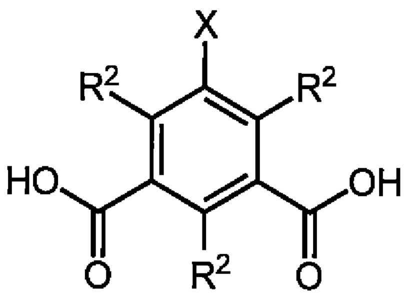

(IV)

wherein in the formula, $R^2$s are each independently a hydrogen atom, a halogen atom or an alkyl group having 1 to 4 carbon atoms, and X is a hydrogen atom, an alkyl group having 1 to 4 carbon atoms, an alkenyl group having 2 to 4 carbon atoms, an alkynyl group having 2 to 4 carbon atoms, an alkoxy group having 1 to 4 carbon atoms, a nitro group, a carboxy group, a hydroxy group or an amino group; a hydroxycarboxylic acid compound represented

by the following general formula (V):

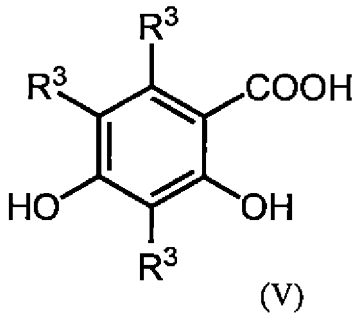

(V)

wherein in the formula, R3s are each independently a hydrogen atom, an alkyl group having 1 to 4 carbon atoms, an alkenyl group having 2 to 4 carbon atoms, an alkynyl group having 2 to 4 carbon atoms or an alkoxy group having 1 to 4 carbon atoms; an organic compound represented by the following general formulas (VI) to (VIII):

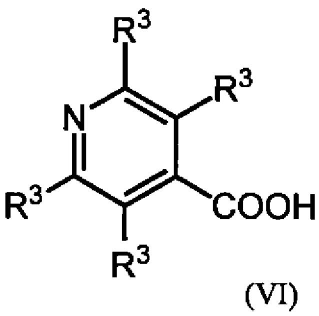

(VI)

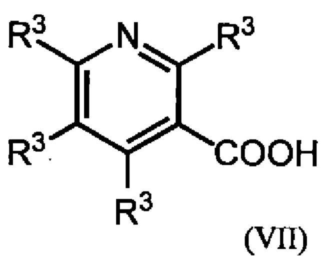

(VII)

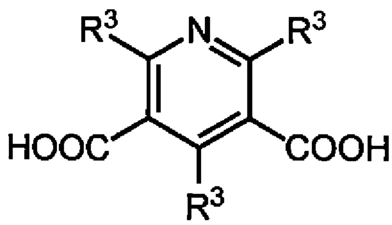

(VIII)

wherein in the formulas, R3s are each independently a hydrogen atom, an alkyl group having 1 to 4 carbon atoms, an alkenyl group having 2 to 4 carbon atoms, an alkynyl group having 2 to 4 carbon atoms or an alkoxy group having 1 to 4 carbon atoms; and an organic compound represented by the following general formulas (IX) to (XII):

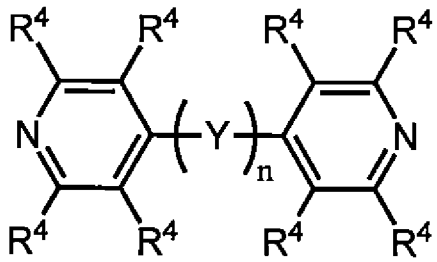

(IX)

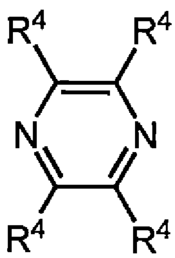

(X)

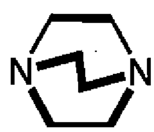

(XI)

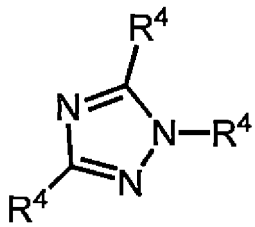

(XII)

wherein in the formulas, Ys are each independently an oxygen atom, a sulfur atom, $-CH_2-$ , -CH(OH)-, -CO-, -NH-, $-C_2N_4-$, -C=C-, $-C_2H_2-$ or $-C_6H_4-$, and $R^4$s are each independently a hydrogen atom, a halogen atom, an alkyl group having 1 to 4 carbon atoms, an alkoxy group having 1 to 4 carbon atoms, a formyl group, an acyloxy group having 1 to 4 carbon atoms, an alkoxycarbonyl group having an alkoxy group having 1 to 4 carbon atoms, a nitro group, a cyano group, a carboxy group, an amino group, a monoalkylamino group having 1 to 4 carbon atoms, a dialkylamino group having alkyl groups having 1 to 4 carbon atoms, or an acylamino group having 1 to 4 carbon atoms, and n is an integer from 0 to 3.

[ADVANTAGEOUS EFFECTS OF INVENTION]

In a step of recovering an unreacted olefin by a pressure swing adsorption method in a chemical reaction

process using the olefin as a raw material, the unreacted olefin can be desorbed from a separation agent at a relatively high desorption operation pressure, more preferably a desorption operation pressure equal to or higher than normal pressure, and the olefin can be recovered, and a capital investment for depressurization and an energy cost of operation can be suppressed.

BRIEF DESCRIPTION OF DRAWINGS

FIG. 1 is a schematic diagram of an adsorption isotherm of a gate-open type adsorbent.
FIG. 2 is a schematic diagram of a differential equation of an adsorption isotherm of a gate-open type adsorbent.
FIG. 3 is a schematic diagram of a chemical reaction process using an olefin as a raw material.
FIG. 4 is a schematic diagram of an olefin recovery apparatus.
FIG. 5 is an adsorption desorption isotherm of ethylene at 25°C of the metal complex (1) of Example 1.
FIG. 6 is an adsorption desorption isotherm of ethylene at 25°C of the separation agent (1) of Example 1.
FIG. 7 is a graph obtained by differentiating the adsorption desorption isotherm of ethylene at 25°C of the separation agent (1) of Example 1 with respect to pressure (P).
FIG. 8 is an adsorption desorption isotherm of ethylene at 25°C of the metal complex (2) of Example 2.
FIG. 9 is an adsorption desorption isotherm of ethylene at 25°C of the separation agent (2) of Example 2.
FIG. 10 is a graph obtained by differentiating the adsorption desorption isotherm of ethylene at 25°C of the separation agent (2) of Example 2 with respect to pressure (P).
FIG. 11 is an adsorption desorption isotherm of ethylene at 25°C of the metal complex (3) of Comparative Example 1.
FIG. 12 is an adsorption desorption isotherm of ethylene at 25°C of the separation agent (3) of Comparative Example 1.
FIG. 13 is a graph obtained by differentiating the adsorption desorption isotherm of ethylene at 25°C of the separation agent (3) of Comparative Example 1 with respect to pressure (P).
FIG. 14 is an X-ray powder diffraction pattern of the metal complex (1) of Example 1.
FIG. 15 is an X-ray powder diffraction pattern of the metal complex (2) of Example 2.
FIG. 16 is an X-ray powder diffraction pattern of the metal complex (3) of Comparative Example 1.

DESCRIPTION OF EMBODIMENTS

The present invention will now be described in detail, but the following description should not be construed as disclosing all embodiments of the present invention and all advantages thereof.

1. Method for recovering olefin

A separation agent (adsorbent) used in the method for recovering an olefin according to one embodiment is classified into a gate-open type adsorbent, and the relationship between a pressure (P) and an adsorbed amount (A) in an adsorption isotherm thereof will be described with reference to the schematic diagram of FIG. 1. An equilibrium adsorbed amount at an adsorption operation pressure P1 in the isotherm at the time of adsorption (solid squares in the FIG.) is A1, and an equilibrium adsorbed amount at a desorption operation pressure P2 in the isotherm at the time of desorption (black-framed squares in the FIG.) is A2. At this time, the isotherm at the time of adsorption and the isotherm at the time of desorption may completely overlap each other, or the isotherm at the time of desorption may shift to the low pressure side to exhibit hysteresis.

Next, when the adsorbed amount (A) is expressed as a function of the pressure (P), A = f(P), the relationship between a value (dA/dP) obtained by differentiating A with respect to P and the pressure (P) will be described with reference to the schematic diagram of FIG. 2. In dA/dP at the time of adsorption (solid squares in the FIG.), there is a local maximum value at P3 in the lower pressure side than the adsorption operation pressure P1, and in dA/dP at the time of desorption (black-framed squares in the FIG.), there is a local maximum value at P4 in the higher pressure side than the desorption operation pressure P2. At this time, the pressure P3 corresponding to the local maximum value at the time of adsorption and the pressure P4 corresponding to the local maximum value at the time of desorption may completely overlap each other, or the pressure P4 may shift to the lower pressure side.

The method for recovering an olefin according to one embodiment is characterized in that, a pressure swing adsorption method is used in a stream of a chemical reaction process using an olefin as a raw material, and on the basis of an adsorption isotherm indicating an adsorption pressure (P) and an olefin adsorbed amount (A), a metal complex in which a pressure P3 corresponding to a local maximum value at the time of adsorption and a pressure P4 corresponding to a local maximum value at the time of desorption exist between an adsorption operation pressure P1 and a desorption

operation pressure P2, with respect to a value (dA/dP) obtained by differentiating A with respect to P, in the case that the adsorbed amount (A) is expressed as a function of the adsorption pressure (P), A = f(P), is used as a separation agent.

In the adsorption isotherm at the time of adsorption operation of the metal complex, it is preferable that an olefin adsorbed amount A1 at the adsorption operation pressure P1 and an olefin adsorbed amount A2 at the desorption operation pressure P2 satisfy the relationship that a value of (A1 - A2) × P2 / ((P1 - P2) × A2) is 0.4 or more, more preferably satisfy the relationship that the value of (A1 - A2) × P2 / ((P1 - P2) × A2) is 0.5 or more, and still more preferably satisfy the relationship that the value of (A1 - A2) × P2 / ((P1 - P2) × A2) is 2.0 or more, from the viewpoint of separation efficiency. The above equation is obtained by rewriting the following equation.

$$\frac{\dfrac{A1 - A2}{P1 - P2}}{\dfrac{A2}{P2}} \geqq 0.4$$

A suitable olefin (gas) to be recovered is a hydrocarbon having 2 to 4 carbon atoms, and examples thereof include ethane, ethylene, acetylene, propane, propylene, methylacetylene (1-propyne), n-butane, isobutane, 1-butene, trans-2-butene, isobutene, and 1,3-butadiene. Ethylene is particularly preferable.

The chemical reaction process is not particularly limited as long as it uses an olefin as a raw material, and preferably any one of a vinyl acetate production process, an ethyl acetate production process, an allyl acetate production process, a polyethylene production process, and a polypropylene production process.

2. Metal complex

The metal complex, which is a separation agent used in the method for recovering an olefin according to one embodiment, and in which, on the basis of an adsorption isotherm indicating an adsorption pressure (P) and an olefin adsorbed amount (A), a pressure P3 corresponding to a local maximum value at the time of adsorption and a pressure P4 corresponding to a local maximum value at the time of desorption exist between an adsorption operation pressure P1 and a desorption operation pressure P2, with respect to a value (dA/dP) obtained by differentiating A with respect to P, in the case that the adsorbed amount (A) is expressed as a function of the adsorption pressure (P), A = f(P), is preferably a metal complex formed of a metal ion and an organic ligand capable of bonding with the metal ion. Such a metal complex has a porous structure. The porous structure includes molecular-level pores which can accommodate gas molecules.

It is preferable that the adsorption isotherm of an olefin of the aforementioned metal complex be an adsorption isotherm in which the adsorbed amount rapidly increases when the pressure exceeds a predetermined pressure, while the metal complex is less likely to exhibit adsorption up to the predetermined pressure, and with respect to the desorption, an adsorption isotherm in which the adsorbed amount rapidly decreases, i.e., the desorption amount rapidly increases, when the pressure is less than a predetermined pressure, while the metal complex is less likely to exhibit desorption above the predetermined pressure, as described in Non-Patent Literature 1. The adsorption operation pressure P1 is preferably in a pressure range corresponding to a region in which the adsorbed amount is sufficiently high, and the desorption operation pressure P2 is preferably in a pressure range corresponding to a region in which the adsorbed amount is sufficiently reduced. It is more preferable that P2 be equal to or higher than normal pressure, i.e., equal to or higher than 1 atm, which is a normal atmospheric pressure.

The pores of the aforementioned metal complex preferably have a flexible structure capable of adsorbing a gas while accompanied by a change in the structure or size thereof caused by an external stimulus, such as pressure. The size of the pores of the porous metal complex is not particularly limited, and for example, preferably 2 Å to 50 Å, more preferably 2 Å to 30 Å, and more preferably 2 Å to 20 Å.

2-1. Metal ion

The metal ion constituting the metal complex is not particularly limited as long as it can form pores capable of accommodating a specific molecule by organization with an organic ligand. Preferred examples thereof include a cation of at least one metal selected from the group consisting of magnesium, calcium, aluminum, vanadium, manganese, iron, cobalt, nickel, copper, zinc, cadmium, lead, and palladium. An ion of at least one metal selected from the group consisting of magnesium, aluminum, copper, and zinc is more preferable. Copper and zinc are most preferable.

2-2. Organic ligand

The organic ligand constituting the metal complex is not particularly limited as long as it is an organic compound which has two or more sites capable of coordination bonding with the metal ion in the molecule and can constitute a porous structure having a plurality of pores capable of accommodating a specific molecule by organization with the metal ion, and is preferably at least one organic compound selected from the group consisting of the following (1) to (3).

Organic ligand (1): An organic compound capable of bidentate coordination to the metal ion, and having two or more carboxy groups and/or hydroxy groups in the molecule, and having no heterocycle

Organic ligand (2): A saturated or unsaturated, monocyclic or polycyclic heterocyclic compound capable of bidentate coordination to the metal ion, and having a carboxy group or a hydroxy group, and having one heteroatom selected from N, O or S in the ring

Organic ligand (3): A saturated or unsaturated, monocyclic or polycyclic heterocyclic compound capable of bidentate coordination to the metal ion, and having two or more heteroatoms selected from the group consisting of N, O and S in one or more of its rings

<Organic ligand (1): An organic compound capable of bidentate coordination to the metal ion, and having two or more carboxy groups and/or hydroxy groups in the molecule, and having no heterocycle>

Examples of the organic ligand (1) include an alkylene dicarboxylic acid compound having 4 to 20 carbon atoms (the number of carbon atoms includes a carbon atom constituting a carboxy group), an alkenylene dicarboxylic acid compound having 4 to 20 carbon atoms (the number of carbon atoms includes a carbon atom constituting a carboxy group), a dicarboxylic acid compound represented by the following general formulas (I) to (IV), and a hydroxycarboxylic acid compound represented by the following general formula (V).

The number of carbon atoms of the alkylene dicarboxylic acid compound having 4 to 20 carbon atoms (the number of carbon atoms includes a carbon atom constituting a carboxy group) is preferably 4 to 10, and more preferably 4 to 6, from the viewpoint of the pore size of the obtained complex. Specific examples thereof include succinic acid, glutaric acid, and adipic acid. Among these, succinic acid is preferable.

The number of carbon atoms of the alkenylene dicarboxylic acid compound having 4 to 20 carbon atoms (the number of carbon atoms includes a carbon atom constituting a carboxy group) is preferably 4 to 10, and more preferably 4 to 6, from the viewpoint of the pore size of the obtained complex. Specific examples thereof include fumaric acid, glutaconic acid, and muconic acid (hexenedicarboxylic acid).

The dicarboxylic acid compounds represented by the general formulas (I) to (III) are represented by the following chemical formulas.

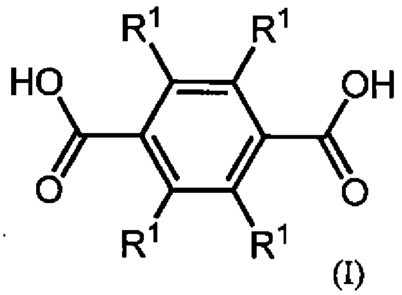

(I)

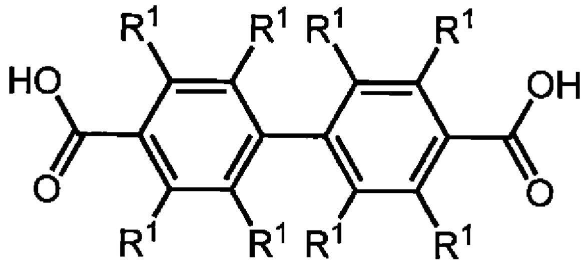

(II)

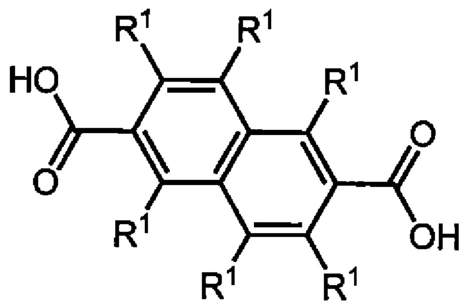

(III)

In the formulas (I) to (III), $R^1$s are each independently a hydrogen atom, a halogen atom, an alkyl group having 1 to 4 carbon atoms, an alkoxy group having 1 to 4 carbon atoms, a formyl group, an acyloxy group having 1 to 4 carbon atoms, an alkoxycarbonyl group having an alkoxy group having 1 to 4 carbon atoms, a nitro group, a cyano group, a carboxy group, an amino group, a monoalkylamino group having 1 to 4 carbon atoms, a dialkylamino group having alkyl groups having 1 to 4 carbon atoms, or an acylamino group having 1 to 4 carbon atoms, and two or more of $R^1$s may be condensed to form a ring. In the aforementioned dialkylamino group, two alkyl groups may be identical to or different from each other.

As the halogen atom, a fluorine atom and a chlorine atom are preferable.

The alkyl group having 1 to 4 carbon atoms may be any of linear, branched or cyclic, and examples thereof include a methyl group, an ethyl group, a propyl group, an isopropyl group, and a butyl group. Examples of the alkoxy group having 1 to 4 carbon atoms include a methoxy group, an ethoxy group, a propoxy group, and a butoxy group. Examples of the acyloxy group having 1 to 4 carbon atoms include those in which a linear or branched alkyl group having 1 to 4 carbon atoms is substituted, such as an acetoxy group, a propionyloxy group, and an isopropionyloxy group. Examples of the alkoxycarbonyl group having an alkoxy group having 1 to 4 carbon atoms include those in which a linear or branched alkyl group having 1 to 4 carbon atoms is substituted, such as a methoxycarbonyl group, an ethoxycarbonyl group, a propoxycarbonyl group, an isopropoxycarbonyl group, and a butoxycarbonyl group. Examples of the monoalkylamino group having 1 to 4 carbon atoms include those in which a linear or branched alkyl group having 1 to 4 carbon atoms is substituted, such as a methylamino group, an ethylamino group, a propylamino group, an isopropylamino group, a butylamino group, and an isobutylamino group. Examples of the dialkylamino group having alkyl groups having 1 to 4 carbon atoms include those in which linear or branched alkyl groups having 1 to 4 carbon atoms are substituted, such as a dimethylamino group, a diethylamino group, a dipropylamino group, a diisopropylamino group, and di-sec-butylamino group. Examples of the acylamino group having 1 to 4 carbon atoms include those in which a linear or branched alkyl group having 1 to 4 carbon atoms is substituted, such as an acetylamino group, and a propionylamino group. Among these, a hydrogen atom is preferable as $R^1$.

The dicarboxylic acid compound represented by the general formula (IV) is represented by the following chemical formula.

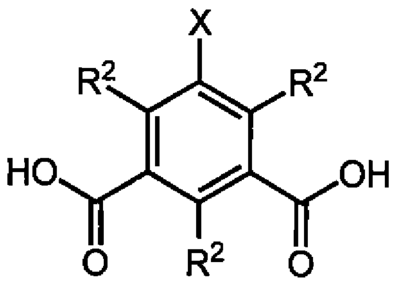

(IV)

In the formula (IV), $R^2$s are each independently a hydrogen atom, a halogen atom or an alkyl group having 1 to 4 carbon atoms, and X is a hydrogen atom, an alkyl group having 1 to 4 carbon atoms, an alkenyl group having 2 to 4 carbon atoms, an alkynyl group having 2 to 4 carbon atoms, an alkoxy group having 1 to 4 carbon atoms, a nitro group, a carboxy group, a hydroxy group or an amino group. The alkenyl group having 2 to 4 carbon atoms may be any of linear, branched or cyclic, and examples thereof include a vinyl group, an allyl group, and a crotyl group, and the alkynyl group having 2 to 4 carbon atoms may be either linear or branched, and examples thereof include an ethynyl group, a propargyl group, and a butynyl group. Specific examples of other $R^2$s and X are the same as those described in the aforementioned $R^1$. Among these, it is preferable that $R^2$ be a hydrogen atom. As X, a hydrogen atom, a methyl group, a nitro group, and a carboxy group are preferable from the viewpoint of raw material cost.

The hydroxycarboxylic acid compound represented by the general formula (V) is represented by the following chemical formula.

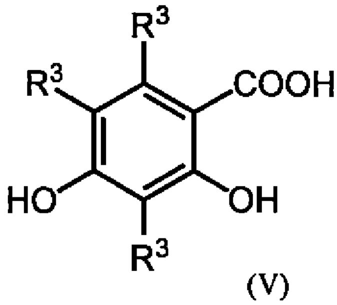

(V)

In the formula (V), $R^3$s are each independently a hydrogen atom, an alkyl group having 1 to 4 carbon atoms, an alkenyl group having 2 to 4 carbon atoms, an alkynyl group having 2 to 4 carbon atoms, or an alkoxy group having 1 to 4 carbon atoms. Specific examples of $R^3$ are the same as those described in the aforementioned $R^1$ and $R^2$. Among these, it is preferable that $R^3$ be a hydrogen atom.

As the organic ligand (1), fumaric acid, terephthalic acid, isophthalic acid, 5-nitroisophthalic acid, and methylisophthalic acid are preferable.

<Organic ligand (2): A saturated or unsaturated, monocyclic or polycyclic heterocyclic compound capable of bidentate coordination to the metal ion, and having a carboxy group or a hydroxy group, and having one heteroatom selected from N, O or S in the ring>

Examples of the organic ligand (2) include organic compounds represented by the following general formulas (VI) to (VIII).

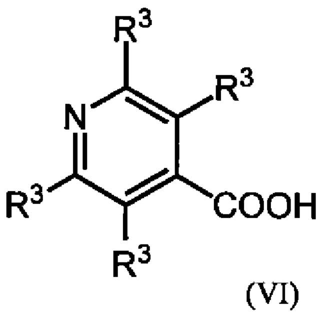

(VI)

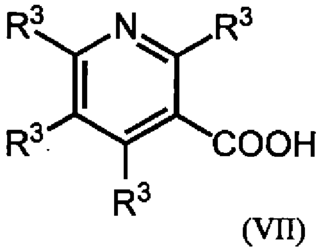

(VII)

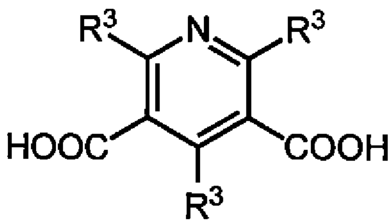

(VIII)

In the formulas (VI) to (VIII), R3s are each independently a hydrogen atom, an alkyl group having 1 to 4 carbon atoms, an alkenyl group having 2 to 4 carbon atoms, an alkynyl group having 2 to 4 carbon atoms, or an alkoxy group having 1 to 4 carbon atoms. Specific examples of $R^3$ are the same as those described in the aforementioned $R^1$ and $R^2$. Among these, it is preferable that $R^3$ be a hydrogen atom.

As the organic ligand (2), pyridinedicarboxylic acid, isonicotinic acid, and nicotinic acid are preferable.

<Organic ligand (3): A saturated or unsaturated, monocyclic or polycyclic heterocyclic compound capable of bidentate coordination to the metal ion, and having two or more heteroatoms selected from the group consisting of N, O and S in one or more of its rings>

Examples of the organic ligand (3) include organic compounds represented by the following general formulas (IX) to (XII).

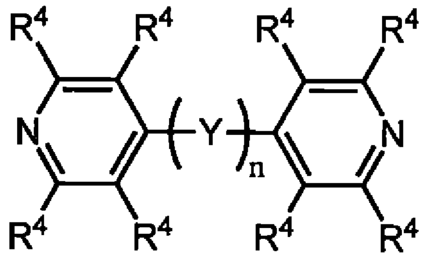
(IX)

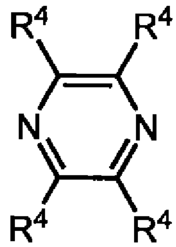
(X)

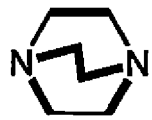
(XI)

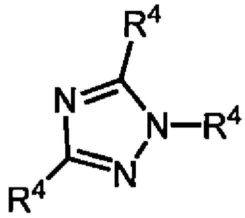
(XII)

In the formulas (IX), (X) and (XII), Ys are each independently an oxygen atom, a sulfur atom, - $CH_2$-, -CH(OH)-, -CO-, -NH-, -$C_2N_4$- (1,2,4,5-tetrazine-3,6-diyl group), -C=C-, -$C_2H_2$-, or - $C_6H_4$-, and preferably -$CH_2$-, -C=C-, -$C_2H_2$-, or -$C_6H_4$-. $R^4$s are each independently a hydrogen atom, a halogen atom, an alkyl group having 1 to 4 carbon atoms, an alkoxy group having 1 to 4 carbon atoms, a formyl group, an acyloxy group having 1 to 4 carbon atoms, an alkoxycarbonyl group having an alkoxy group having 1 to 4 carbon atoms, a nitro group, a cyano group, a carboxy group, an amino group, a monoalkylamino group having 1 to 4 carbon atoms, a dialkylamino group having alkyl groups having 1 to 4 carbon atoms, or an acylamino group having 1 to 4 carbon atoms. Specific examples of $R^4$ are the same as those described in the aforementioned $R^1$. n is an integer from 0 to 3, and preferably 0, 1 or 2.

As the organic ligand (3), pyrazine and derivatives thereof, 4,4'-bipyridine, 1,2-di(4-pyridyl)ethane, 1,2-di(4-pyridyl)ethylene, and 1,2-di(4-pyridyl)acetylene are preferable.

The aforementioned organic ligands may be used alone, or in combination of two or more thereof, and may be suitably selected depending on the type of olefin to be adsorbed. Among the aforementioned organic compounds, the alkylene dicarboxylic acid compound having 4 to 20 carbon atoms, and the organic compounds represented by the general formula (I), the general formula (IV), the general formula (IX) and the general formula (X) are preferable. More preferred examples thereof include fumaric acid, terephthalic acid and derivatives thereof, isophthalic acid and derivatives thereof, pyrazine and derivatives thereof, 4,4'-bipyridine, 1,2-di(4-pyridyl)ethane, 1,2-di(4-pyridyl)ethylene, and 1,2-di(4-pyridyl)acetylene, and still more preferred examples thereof include 1,3,5-benzenetricarboxylic acid, 5-nitroisophthalic acid, pyrazine, 2,3-pyrazinecarboxylic acid, 1,2-di(4-pyridyl)ethane, and 1,2-di(4-pyridyl)ethylene.

There is no particular limitation on a combination of two or more of the organic ligands used. For example, a combination of organic ligands selected from each of the aforementioned organic ligand (1) and organic ligand (3), and a combination of two or more organic ligands selected from the aforementioned organic ligand (3) are preferable, and more preferred examples thereof include a combination of organic compounds represented by the general formula (I) and the general formula (IX), a combination of organic compounds represented by the general formula (IV) and the general formula (IX), a combination of organic compounds represented by the general formula (X) and the general formula (IX), and a combination of two or more organic compounds represented by the general formula (X), and still more preferred examples thereof include a combination of 5-nitroisophthalic acid, 1,2-di(4-pyridyl)ethane and 1,2-di(4-pyridyl)ethylene, and a combination of 2,3-pyrazinecarboxylic acid and pyrazine.

2-3. Method for producing metal complex

The metal complex can be obtained by dissolving a metal raw material selected from the group consisting of a metal salt of the aforementioned metal, such as a nitrate, a sulfate, a formate, an acetate, a carbonate, a hydrochloride, a hydrobromide, a tetrafluoroborate, and a hexafluorophosphate, a hydroxide thereof, and an oxide thereof, and the aforementioned organic ligand in water or an organic solvent, and reacting them for several hours to several days. As the organic solvent, any solvent may be used as long as the aforementioned metal salt and the organic ligand are dissolved therein, and for example, methanol, ethanol, propanol, diethyl ether, tetrahydrofuran, hexane, cyclohexane, benzene, toluene, methylene chloride, chloroform, acetone, ethyl acetate, acetonitrile, dimethyl sulfoxide (DMSO), N,N-dimethylformamide (DMF), water or a mixed solvent of two or more thereof can be used. The reaction conditions are not particularly limited and may be suitably adjusted according to the degree of the reaction progress, and for example, the reaction temperature is preferably set at room temperature (25°C) to 150°C. Further, the aforementioned reaction may be carried out under pressure.

As an example of the manufacturing method, there is a method using a wet grinder. For example, an apparatus can be charged with a dicarboxylic acid compound (I), a metal salt, two or more of dipyridyl compounds (IX), a solvent, and a ball for grinding, and the reaction can proceed while performing a grinding operation.

In the aforementioned reaction, it is preferable that the raw materials be reacted in the wet grinder while grinding the produced metal complex which is being precipitated as a crystal to complete the reaction.

Examples of the apparatus used for wet grinding include a grinder, such as a ball mill, a rod mill, and a bead mill; and a kneading machine, such as a kneader, and a twin screw extruder. The use of a grinder is advantageous from the viewpoint of an adsorption rate, since a metal complex having a smaller particle size can be obtained as compared with a conventional hydrothermal synthesis method, etc. Further, since the synthesis is completed in about several minutes to several hours, the production time can be significantly shortened.

3. Separation agent for recovering olefin

Examples of the form of the metal complex which can be used as a separation agent for recovering an olefin include various aspects, such as granular, powdery, fibrous, film-like, and plate-like, and the metal complex is preferably in the form of powder. The metal complex having an average particle diameter of 1 μm to 500 μm, and more preferably 5 μm to 100 μm, is preferably used. In the present disclosure, the average particle diameter refers to a diameter at 50% of the number cumulative distribution (median diameter), and is measured by a laser diffraction/scattering particle size distribution analyzer.

As the separation agent, the aforementioned metal complex may be used as it is, or in the form of a molded article formed by a suitable method.

The amount of the metal complex contained in the separation agent is preferably 50% by mass to 97% by mass. Considering the adsorption performance and the productivity of the separation agent, the content of the metal complex is more preferably 70% by mass to 97% by mass. When the content of the metal complex is 50% by mass or more, the adsorption efficiency of a hydrocarbon gas per unit mass can be increased, and on the other hand, when the content is 97% by mass or less, the productivity of the separation agent can be improved, or a required strength for the separation agent can be obtained.

The separation agent may contain, if necessary, an additive, such as a polymer binder component, and a lubricant, in addition to the metal complex.

The polymer binder component is a component which serves as a binder for binding metal complex particles. The polymer binder component is preferably at least one selected from the group consisting of an ethylenic hydrocarbon polymer, a polyester, a polyamide, and a polyether, and particularly preferably a copolymer of (meth)acrylic acid and an ester of (meth)acrylic acid having 1 to 10 carbon atoms. Note that "(meth)acrylic acid" refers to "acrylic acid" or "methacrylic acid".

Specific examples of the ethylenic hydrocarbon polymer include partially saponified polyvinyl acetate, partially saponified polyvinyl butyrate, a polyvinyl butyral resin, a copolymer of methyl methacrylate and hydroxyethyl methacrylate, a copolymer of methyl methacrylate and methacrylic acid, a copolymer of ethyl methacrylate and methacrylic acid, a copolymer of propyl methacrylate and methacrylic acid, a copolymer of butyl methacrylate and methacrylic acid, a copolymer of amyl methacrylate and methacrylic acid, a copolymer of 2-ethylhexyl methacrylate and methacrylic acid, a copolymer of methyl acrylate and hydroxyethyl acrylate, a copolymer of methyl acrylate and acrylic acid, a copolymer of ethyl acrylate and acrylic acid, a copolymer of propyl acrylate and acrylic acid, a copolymer of butyl acrylate and acrylic acid, a partially saponified product of a copolymer of ethylene and vinyl acetate, a copolymer of ethylene and acrylic acid, a copolymer of ethylene and methacrylic acid, a copolymer of styrene and acrylic acid, a copolymer of styrene and methacrylic acid, a copolymer of methyl methacrylate and glycidyl methacrylate, a copolymer of methyl acrylate and glycidyl acrylate, a copolymer of ethylene and glycidyl methacrylate, a copolymer of methyl methacrylate and dimethylaminoethyl methacrylate, a copolymer of methyl acrylate and dimethylaminoethyl acrylate, a copolymer of ethylene and dimethylaminoethyl methacrylate, a copolymer of methyl methacrylate and styrenesulfonic acid, and a copolymer of methyl methacrylate and vinylsulfonic acid.

Examples of the polyester include polyethylene terephthalate, polybutylene terephthalate, and polylactic acid. Examples of the polyamide include 6-nylon (registered trademark), and 6,6-nylon (registered trademark). Examples of the polyether include polyethylene glycol, polypropylene glycol, polyoxymethylene, and polyphenylene ether. These polymers have functional groups at their terminals. When the amount of functional groups is less than an amount required for combining with the metal complex, a functional group can be introduced into the polymer by copolymerizing (co-condensating) a monomer having a functional group on a side chain thereof.

The amount of the polymer binder component contained in the separation agent is preferably 3% by mass to 20% by mass, more preferably 5% by mass to 15% by mass, and still more preferably 5% by mass to 12% by mass, with respect to 100% by mass of the total of the components of the separation agent.

The lubricant is an additive to prevent tableting failure during tableting molding. The lubricant is not particularly limited as long as it can prevent tableting failure, such as capping, during tableting. Specific examples of the lubricant include graphite, boron nitride, stearic acid, and stearic acid esters.

The amount of the lubricant contained in the separation agent is preferably 0.1% by mass to 5% by mass, and more preferably from 1% by mass to 4% by mass, with respect to 100% by mass of the total of the components of the separation agent.

It is preferable that the size of the separation agent be 1.0 mm to 10.0 mm. The size is more preferably 3.0 mm to 7.0 mm, and still more preferably 4.0 mm to 6.0 mm. When the size of the separation agent is 1.0 mm or more, it is possible to increase the strength of the molded body or to achieve satisfactory productivity. When the size of the separation agent is 10.0 mm or less, the diffusivity of an olefin inside the molded body can be increased to improve the amount of adsorption per time. In the present disclosure, the size of the separation agent refers to the maximum diameter in a predetermined direction (Krummbein diameter).

4. Method for producing separation agent for recovering olefin

A method for producing a separation agent for recovering an olefin is not particularly limited, and examples thereof include a tableting molding method. When a pellet is produced by a tableting molding method, the metal complex and other components are mixed at a predetermined blending ratio and granulated. Then, the obtained granulated product, and the lubricant and other components which are optionally used are mixed at a predetermined blending ratio and tableted by a tableting apparatus under pressure.

5. Adsorption desorption operation

As a method for recovering an olefin using a separation agent for recovering an olefin, a pressure swing adsorption method is preferable.

The pressure swing adsorption method includes an adsorption step in which a mixed gas containing an olefin to be recovered is brought into contact with the aforementioned separation agent for recovering an olefin at an adsorption operation pressure P1 to selectively adsorb the olefin. In the adsorption step, the pressure of a packed tower filled with the aforementioned separation agent is increased to the adsorption operation pressure P1, and the mixed gas containing the target olefin is supplied, whereby the target olefin is adsorbed and concentrated on the separation agent, and the remaining gas of the mixed gas in which the target olefin is reduced is discharged.

The pressure swing adsorption method includes, after the aforementioned adsorption step, a desorption step in which the pressure is changed to a desorption operation pressure P2 to desorb the olefin adsorbed on the separation agent. In the desorption step, the pressure of the packed tower filled with the aforementioned separation agent is decreased to the desorption operation pressure P2, whereby the target olefin is desorbed from the separation agent and a gas in which the target olefin is concentrated is discharged.

In the pressure swing adsorption method, the adsorption temperature is determined by the olefin to be recovered and the metal complex used in the separation agent, and is preferably 173 to 373 K, more preferably 223 to 353 K, and most preferably 273 to 333 K.

In the pressure swing adsorption method, the adsorption operation pressure P1 and the desorption operation pressure P2 are not particularly limited, and it is preferable that the difference between an adsorbed amount A1 at P1 and an adsorbed amount A2 at P2 be large. The adsorption operation pressure P1 is preferably 10 kPaG to 10 MPaG (110 kPaA to 10.1 MPaA), more preferably 50 kPaG to 3 MPaG (150 kPaA to 3.1 MPaA), and particularly preferably 100 kPaG to 1 MPaG (200 kPaA to 1.1 MPaA). The desorption operation pressure P2 is preferably - 100 kPaG to 1 MPaG (≈ 0 kPaA to 1.1 MPaA), more preferably -50 kPaG to 500 kPaG (50 kPaA to 600 kPaA), and most preferably normal pressure (0 kPaG) to 100 kPaG (100 kPaA to 200 kPaA). "kPaG" refers to a gauge pressure, and "kPaA" refers to an absolute pressure.

A schematic diagram of an olefin recovery apparatus is shown in FIG. 4 as a preferred embodiment. In FIG. 4, an adsorption tower 4 is filled with a separation agent containing the aforementioned metal complex, one end of which is connected to a raw material gas supply line via a valve V1, and a line for an olefin concentrated gas via a valve V3, and the other end of which is connected to a line for a permeated gas via a V2, and a buffer tank 1, a buffer tank 2, and a buffer tank 3 are respectively connected to the valves V1 to V3, in order to suppress pressure fluctuation or flow rate pulsation. The valves V1 to V3 are adapted to be controlled in terms of their operations by a control means. The pressure swing adsorption method is characterized by not necessarily requiring a compressor or pressure reducer generally used.

When the separation apparatus shown in FIG. 4 is used for recovering an olefin, for example, it is controlled as follows. First, a raw material gas is introduced into the adsorption tower 4 through the valve V1 via the buffer tank 1, and the pressure in the adsorption tower 4 is increased in a state in which the valve V2 is closed, whereby a target olefin is selectively adsorbed on a separation agent containing the aforementioned metal complex up to an adsorption operation pressure P1. Thereafter, by opening the valve V2, a gas in which the concentration of the target olefin is reduced is discharged through the valve V2 via the buffer tank 2 as a permeated gas.

Next, by closing the valves V1 and V2 and opening the valve V3, the pressure in the adsorption tower 4 is reduced to a desorption operation pressure P2, whereby a gas in which the target olefin is concentrated is desorbed from the separation agent containing the aforementioned metal complex, and is discharged as an olefin concentrated gas through the buffer tank 3.

EXAMPLES

The present invention will be described in more detail below with reference to examples, but the present invention is not limited by the following examples, and it is of course possible to carry out the present invention with appropriate modifications to the extent that it can conform to the spirit of the present disclosure, and any of them is included in the technical scope of the present invention.

1. Production of separation agent for recovering olefin

A metal complex and a polymer binder solution were mixed and kneaded in a mortar, and pulverized while air-drying, and a mixture passed through a 0.75 mm mesh sieve was obtained as a granulated product. A mixture obtained by adding thereto graphite as a lubricant in a necessary amount and well mixing them was tableted by using a tableting apparatus at a tableting pressure of 600 MPa to produce a cylindrical molded body (pellet) having a thickness of about 4 mm and a diameter of 3 mm. The obtained molded body was dried under reduced pressure for 6 hours or more at 120°C to remove adsorbed water, etc., and then subjected to each test.

2. Evaluation method

2-1. Adsorption desorption isotherm

The molded bodies (pellets) used in Examples and Comparative Examples were measured at 25°C by a volumetric method using a gas adsorption measuring apparatus (BELSORP-HP (registered trademark) manufactured by Japan Bel Corp.) to prepare adsorption desorption isotherms. In the measurement, the amount of a porous metal complex in the sample was 0.3 g to 0.5 g with respect to all of the samples.

2-2. Separation test

Each of the molded bodies (pellets) obtained in Examples and Comparative Examples was charged in an adsorption tower equipped with valves, buffer tanks, and lines as shown in FIG. 4. A mixed gas containing an olefin was supplied so that an adsorption operation pressure P1 was 700 kPaA and a desorption operation pressure P2 was 120 kPaA, and the valves were controlled to obtain a permeated gas and an olefin concentrated gas. Each gas was sampled and analyzed using gas chromatography.

Analysis conditions:

Apparatus: Agilent 490 micro GC
Column: Porapac Q, 10 m
Carrier: He
Detector: TCD

Example 1

Synthesis of metal complex (1): $[Zn(NO_2\text{-ip})(bpe)_{0.8}(bpa)_{0.2}]$

Zinc oxide (0.41 g, 5.0 mmol, 1 eq.), 5-nitroisophthalic acid (1.07 g, 5.0 mmol, 1.0 eq.), 1,2-di(4-pyridyl)ethane (0.18 g, 1.0 mmol, 0.2 eq.), 1,2-di(4-pyridyl)ethylene (0.73 g, 4.0 mmol, 0.8 eq.), distilled water (5 mL), and zirconia balls (3 mmcp, 25 g) were added to a zirconia container (45 mL), and wet ground (using a Fritsch Classic Line P-7) for 1 hour at 400 rpm at ambient temperature (25°C) while reacting them. Thereafter, the content was filtered using a Kiriyama funnel, and the precipitated metal complex was washed with ion exchanged water and ethanol in this order and then dried. 2.09 g of the metal complex was obtained as a white solid (yield: 91%). This was used as a metal complex (1).

The X-ray powder diffraction pattern of the obtained metal complex (1) was measured. The measurement was carried out by using an X-ray diffraction apparatus ("Multiflex" manufactured by Rigaku Corporation), and scanning a range of diffraction angle (2θ) = 3 to 50° at a scanning speed of 30°/min by a symmetrical reflection method. The measurement result is shown in FIG. 14.

Separation agent (1)

87 parts by mass of the metal complex (1), 5 parts by mass of a polymer binder (a copolymer of methacrylic acid and methyl methacrylate, manufactured by Evonik Japan Co., Ltd., Eudragit S100), and 200 parts by mass of

tetrahydrofuran were mixed and kneaded in a mortar, and pulverized while air-drying to obtain a product which passed through a 0.5 mm mesh sieve as a granulated product. Graphite (manufactured by Nippon Graphite Industries, Co., Ltd., ACP) as a lubricant was added thereto and mixed well so that 100 parts by mass of a molded body contained 3 parts by mass of graphite, and a molded body was produced using a tableting apparatus at room temperature. The obtained molded body was dried for 6 hours or more at 120°C as a separation agent (1).

Adsorption desorption isotherm

Adsorption desorption isotherms of ethylene at 25°C were measured with respect to the metal complex (1) and the separation agent (1). The results are shown in FIGs. 5 and 6, respectively. The vertical axis represents the adsorbed amount of the target gas (ethylene) per unit mass. In FIG. 6, the effective adsorbed amounts of ethylene of the separation agent (1) were 35.1 mL(STP: normal state)/g at 700 kPaA during the adsorption operation and 2.5 mL(STP)/g at 120 kPaA during the desorption operation. The value of (A1 - A2) × P2 / ((PI - P2) × A2) was 2.70.

Differentiation with respect to pressure P

FIG. 7 shows the relationship between a value (dA/dP) obtained by differentiating the adsorbed amount (A) with respect to the pressure (P), when the adsorbed amount (A) is expressed as a function of the pressure (P), A = f(P), on the basis of the adsorption isotherm of ethylene obtained with respect to the separation agent (1). A pressure P3 corresponding to the local maximum value at the time of adsorption is about 240 kPaA, a pressure P4 corresponding to the local maximum value at the time of desorption is about 210 kPaA, and when the adsorption operation pressure P1 is 700 kPaA and the desorption operation pressure P2 is 120 kPaA, P3 and P4 exist between P1 and P2.

Separation test

An adsorption tower of a separation experimental apparatus as shown in FIG. 4 was charged with 17.0 g of the separation agent (1). A mixed gas containing 91.7% of ethylene, 3.1% of ethane, and 5.2% of nitrogen (numerical values are based on volume, hereinafter the same also applies to a raw material gas, a permeated gas, and an olefin concentrated gas) was supplied as a raw material gas at a flow rate of 539.5 $cm^3$ per minute (STP). Table 1 shows the switching states and times of the valves V1, V2, and V3 in each step during the experiment. The process proceeded in the order of Step 1, Step 2, Step 3, and Step 4, and returned to Step 1. The duration of each step was set so that the permeated gas was approximately 20% of the raw material gas. When repeated 50 times to 70 times, the flow amounts per cycle were 944 mL of the raw material gas, 198 mL of the permeated gas, and 800 mL of the olefin concentrated gas, respectively. The analytical results of the permeated gas were 82.2% of ethylene, 4.4% of ethane, 13.4% of nitrogen, and those of the olefin concentrated gas were 93.8% of ethylene, 2.7% of ethane, and 3.5% of nitrogen. These are shown in Table 2.

Example 2

Synthesis of metal complex (2): $[Zn(NO_2\text{-ip})(bpe)_{0.7}(bpa)_{0.3}]$

Zinc oxide (0.41 g, 5.0 mmol, 1 eq.), 5-nitroisophthalic acid (1.07 g, 5.0 mmol, 1.0 eq.), 1,2-di(4-pyridyl)ethane (0.27 g, 1.5 mmol, 0.3 eq.), 1,2-di(4-pyridyl)ethylene (0.63 g, 3.5 mmol, 0.7 eq.), distilled water (5 mL), and zirconia balls (3 mmcp, 25 g) were added to a zirconia container (45 mL), and wet ground (using a Fritsch Classic Line P-7) for 1 hour at 400 rpm at ambient temperature (25°C) while reacting them. Thereafter, the content was filtered using a Kiriyama funnel, and the precipitated metal complex was washed with ion exchanged water and ethanol in this order and then dried. 1.99 g of the metal complex was obtained as a white solid (yield: 88%). This was used as a metal complex (2).

The X-ray powder diffraction pattern of the obtained metal complex (2) was measured by the aforementioned method. The measurement result is shown in FIG. 15.

Separation agent (2)

A separation agent (2) was obtained in the same manner as in Example 1, except that the metal complex (2) was used in place of the metal complex (1).

Adsorption desorption isotherm

Adsorption desorption isotherms of ethylene at 25°C were measured with respect to the metal complex (2) and

the separation agent (2). The results are shown in FIGs. 8 and 9, respectively. In FIG. 9, the effective adsorbed amounts of ethylene of the separation agent (2) were 37.0 mL(STP)/g at 700 kPaA during the adsorption operation and 11.5 mL(STP)/g at 120 kPaA during the desorption operation. The value of (A1 - A2) × P2 / ((PI - P2) × A2) was 0.46.

Differentiation with respect to pressure P

FIG. 10 shows the relationship between a value (dA/dP) obtained by differentiating the adsorbed amount (A) with respect to the pressure (P), when the adsorbed amount (A) is expressed as a function of the pressure (P), A = f(P), on the basis of the adsorption isotherm of ethylene obtained with respect to the separation agent (2). A pressure P3 corresponding to the local maximum value at the time of adsorption is about 170 kPaA, a pressure P4 corresponding to the local maximum value at the time of desorption is about 140 kPaA, and when the adsorption operation pressure P1 is 700 kPaA and the desorption operation pressure P2 is 120 kPaA, P3 and P4 exist between P1 and P2.

Separation test

An adsorption tower of a separation experimental apparatus as shown in FIG. 4 was charged with 19.8 g of the separation agent (2). A mixed gas containing 91.9% of ethylene, 3.0% of ethane, and 5.1% of nitrogen was supplied as a raw material gas at a flow rate of 539.5 $cm^3$ per minute (STP). Table 1 shows the switching states and times of the valves V1, V2, and V3 in each step during the experiment. The process proceeded in the order of Step 1, Step 2, Step 3, and Step 4, and returned to Step 1. The duration of each step was set so that the permeated gas was approximately 20% of the raw material gas. When repeated 50 times to 70 times, the flow amounts per cycle were 939 mL of the raw material gas, 188 mL of the permeated gas, and 766 mL of the olefin concentrated gas, respectively. The analytical results of the permeated gas were 81.2% of ethylene, 4.3% of ethane, 14.5% of nitrogen, and those of the olefin concentrated gas were 93.5% of ethylene, 2.6% of ethane, and 3.8% of nitrogen. These are shown in Table 2.

Comparative Example 1

Synthesis of metal complex (3): $[Zn(NO_2\text{-ip})(bpe)_{0.4}(bpa)_{0.6}]$

Zinc oxide (0.41 g, 5.0 mmol, 1 eq.), 5-nitroisophthalic acid (1.07 g, 5.0 mmol, 1.0 eq.), 1,2-di(4-pyridyl)ethane (0.55 g, 3.0 mmol, 0.6 eq.), 1,2-di(4-pyridyl)ethylene (0.36 g, 2.0 mmol, 0.4 eq.), distilled water (5 mL), and zirconia balls (3 mmcp, 25 g) were added to a zirconia container (45 mL), and wet ground (using a Fritsch Classic Line P-7) for 1 hour at 400 rpm at ambient temperature (25°C) while reacting them. Thereafter, the content was filtered using a Kiriyama funnel, and the precipitated metal complex was washed with ion exchanged water and ethanol in this order and then dried. 1.86 g of the metal complex was obtained as a white solid (yield: 82%). This was used as a metal complex (3).

The X-ray powder diffraction pattern of the obtained metal complex (3) was measured by the aforementioned method. The measurement result is shown in FIG. 16.

Separation agent (3)

A separation agent (3) was obtained in the same manner as in Example 1, except that the metal complex (3) was used in place of the metal complex (1).

Adsorption desorption isotherm

Adsorption desorption isotherms of ethylene at 25°C were measured with respect to the metal complex (3) and the separation agent (3). The results are shown in FIGs. 11 and 12, respectively. In FIG. 12, the effective adsorbed amounts of ethylene of the separation agent (3) were 34.5 mL(STP)/g at 700 kPaA during the adsorption operation and 19.1 mL(STP)/g at 120 kPaA during the desorption operation. The value of (A1 - A2) × P2 / ((P1 - P2) × A2) was 0.17.

Differentiation with respect to pressure P

FIG. 13 shows the relationship between a value (dA/dP) obtained by differentiating the adsorbed amount (A) with respect to the pressure (P), when the adsorbed amount (A) is expressed as a function of the pressure (P), A = f(P), on the basis of the adsorption isotherm of ethylene obtained with respect to the separation agent (3). During the adsorption, a lower pressure showed a higher value, and also during the desorption, a lower pressure showed a higher value, and when the adsorption operation pressure P1 is 700 kPaA and the desorption operation pressure P2 is 120 kPaA, there is no pressure corresponding to a local maximum value between P1 and P2.

Separation test

An adsorption tower of a separation experimental apparatus as shown in FIG. 4 was charged with 18.1 g of the separation agent (3). A mixed gas containing 91.7% of ethylene, 3.0% of ethane, and 5.2% of nitrogen was supplied as a raw material gas at a flow rate of 539.5 $cm^3$ per minute (STP). Table 1 shows the switching states and times of the valves V1, V2, and V3 in each step during the experiment. The process proceeded in the order of Step 1, Step 2, Step 3, and Step 4, and returned to Step 1. The duration of each step was set so that the permeated gas was approximately 20% of the raw material gas. When repeated 50 times to 70 times, the flow amounts per cycle were 675 mL of the raw material gas, 137 mL of the permeated gas, and 547 mL of the olefin concentrated gas, respectively. The analytical results of the permeated gas were 87.9% of ethylene, 4.1% of ethane, 8.0% of nitrogen, and those of the olefin concentrated gas were 92.5% of ethylene, 2.8% of ethane, and 4.7% of nitrogen. These are shown in Table 2.

According to the results of the separation test, when an operation was carried out so that the permeated gas was approximately 20% of the raw material gas, the amounts of ethylene recovered per unit weight of the separation agent per cycle were 44.1 mL/cycle/g with respect to the separation agent (1), and 36.2 mL/cycle/g with respect to the separation agent (2), which are larger than 28.0 mL/cycle/g with respect to the separation agent (3), to indicate that the olefin recovery efficiency is excellent.

Further, the calculated ethylene concentration/(ethane concentration + nitrogen concentration) in the permeated gas are 4.6 with respect to the separation agent (1) and 4.3 with respect to the separation agent (2), which are lower than 7.2 with respect to the separation agent (3), to indicate that the ethylene selectivity is also excellent.

From these results, it is clearly understood that a method for recovering an olefin using the metal complex of the present disclosure as a separation agent is excellent.

Table 1

| | | Step 1 | Step 2 | Step 3 | Step 4 |
|---|---|---|---|---|---|
| | | Pressurization | Adsorption | Blowdown | Desorption |
| Valve | V1 | O | O | C | C |
| | V2 | C | C | C | C |
| | V3 | C | C | O | O |
| Duration | Ex. 1 | 20 seconds | 85 seconds | 20 seconds | 120 seconds |
| | Ex. 2 | 20 seconds | 83 seconds | 20 seconds | 120 seconds |
| | Comp. Ex. 1 | 20 seconds | 54 seconds | 20 seconds | 120 seconds |
| Symbol O: open, C: closed | | | | | |

Table 2

| | | Ex. 1 | Ex. 2 | Comp. Ex. 1 |
|---|---|---|---|---|
| Separation agent | | 17.0 g | 19.8 g | 18.1 g |
| Raw material gas | Flow amount | 944 mL | 939 mL | 675 mL |
| | Ethylene conc. | 91.7% | 91.9% | 91.7% |
| | Ethane conc. | 3.1% | 3.0% | 3.0% |
| | Nitrogen conc. | 5.2% | 5.1% | 5.2% |
| Permeated gas | Flow amount | 198 mL | 188 mL | 137 mL |
| | Ethylene conc. | 82.2% | 81.2% | 87.9% |
| | Ethane conc. | 4.4% | 4.3% | 4.1% |
| | Nitrogen conc. | 13.4% | 14.5% | 8.0% |

(continued)

| | | Ex. 1 | Ex. 2 | Comp. Ex. 1 |
|---|---|---|---|---|
| Separation agent | | 17.0 g | 19.8 g | 18.1 g |
| Olefin concentrated gas | Flow amount | 800 mL | 766 mL | 547 mL |
| | Ethylene conc. | 93.8% | 93.5% | 92.5% |
| | Ethane conc. | 2.7% | 2.6% | 2.8% |
| | Nitrogen conc. | 3.5% | 3.8% | 4.7% |

INDUSTRIAL APPLICABILITY

In the method for recovering an olefin according to the present disclosure, in which an unreacted olefin is recovered from a stream of a chemical reaction process using the olefin as a raw material by a pressure swing adsorption method, the olefin can be efficiently recovered using a separation agent capable of desorbing a gas at a relatively high desorption operation pressure, preferably at a pressure equal to or higher than normal pressure, and being reused.

REFERENCE SIGNS LIST

1, 2 and 3: Buffer tank
4: Adsorption tower
V1, V2 and V3: Valves

## Claims

1. A method for recovering an olefin, in which an unreacted olefin is recovered from a stream of a chemical reaction process using the olefin as a raw material by a pressure swing adsorption method, wherein, on the basis of an adsorption isotherm indicating an adsorption pressure (P) and an olefin adsorbed amount (A), a metal complex in which a pressure P3 corresponding to a local maximum value at the time of adsorption and a pressure P4 corresponding to a local maximum value at the time of desorption exist between an adsorption operation pressure P1 and a desorption operation pressure P2, with respect to a value (dA/dP) obtained by differentiating A with respect to P, in the case that the adsorbed amount (A) is expressed as a function of the adsorption pressure (P), A = f(P), is used as a separation agent.

2. The method for recovering an olefin according to claim 1, wherein, in an adsorption isotherm during an adsorption operation of the metal complex, an olefin adsorbed amount A1 at the adsorption operation pressure P1 and an olefin adsorbed amount A2 at the desorption operation pressure P2 satisfy a relationship of $(A1 - A2) \times P2 / ((P1 - P2) \times A2) \geq 0.4$.

3. The method for recovering an olefin according to claim 1 or 2, wherein the desorption operation pressure P2 is equal to or higher than normal pressure.

4. The method for recovering an olefin according to any one of claims 1 to 3, wherein the number of carbon atoms of the olefin is 2 to 4.

5. The method for recovering an olefin according to any one of claims 1 to 4, wherein the olefin is ethylene.

6. The method for recovering an olefin according to any one of claims 1 to 5, wherein the chemical reaction process is any one of a vinyl acetate production process, an ethyl acetate production process, an allyl acetate production process, a polyethylene production process, and a polypropylene production process.

7. The method for recovering an olefin according to any one of claims 1 to 6, wherein the metal complex is composed of a metal ion and an organic ligand, and the organic ligand is at least one organic compound selected from the

group consisting of (1) to (3) below.

(1) An organic compound capable of bidentate coordination to the metal ion, and having two or more carboxy groups and/or hydroxy groups in the molecule, and having no heterocycle
(2) An organic compound capable of bidentate coordination to the metal ion, and having a monocyclic or polycyclic, saturated or unsaturated heterocycle having one heteroatom selected from N, O or S, and a carboxy group or a hydroxy group in the molecule
(3) An organic compound capable of bidentate coordination to the metal ion, and having a monocyclic or polycyclic, saturated or unsaturated heterocycle having two or more heteroatoms selected from the group consisting of N, O and S in the molecule

**8.** The method for recovering an olefin according to any one of claims 1 to 7, wherein the metal complex is composed of a metal ion and an organic ligand, and the organic ligand is one or more organic compounds selected from the group consisting of an alkylene dicarboxylic acid compound having 4 to 20 carbon atoms; an alkenylene dicarboxylic acid compound having 4 to 20 carbon atoms; a dicarboxylic acid compound represented by the following general formulas (I) to (III):

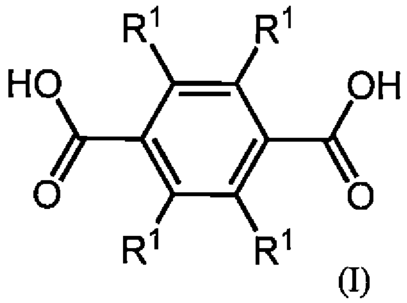

(I)

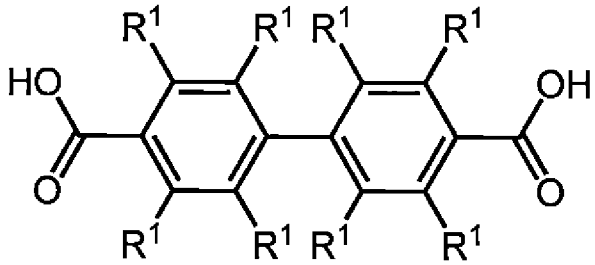

(II)

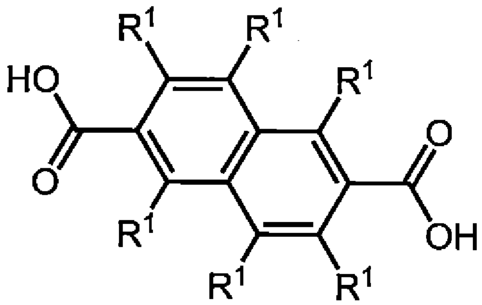

(III)

wherein in the formulas, $R^1$s are each independently a hydrogen atom, a halogen atom, an alkyl group having 1 to 4 carbon atoms, an alkoxy group having 1 to 4 carbon atoms, a formyl group, an acyloxy group having 1 to 4 carbon atoms, an alkoxycarbonyl group having an alkoxy group having 1 to 4 carbon atoms, a nitro group, a cyano group, a carboxy group, an amino group, a monoalkylamino group having 1 to 4 carbon atoms, a dialkylamino group having alkyl groups having 1 to 4 carbon atoms, or an acylamino group having 1 to 4 carbon atoms, and two or more of $R^1$s may be condensed to form a ring; a dicarboxylic acid compound represented by the following general formula (IV):

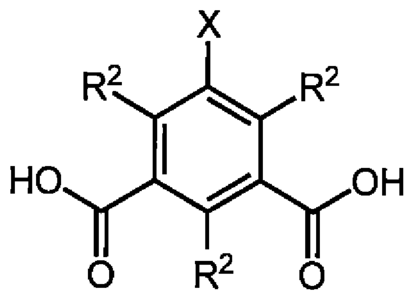

(IV)

wherein in the formula, $R^2$s are each independently a hydrogen atom, a halogen atom or an alkyl group having 1 to 4 carbon atoms, and X is a hydrogen atom, an alkyl group having 1 to 4 carbon atoms, an alkenyl group having 2 to 4 carbon atoms, an alkynyl group having 2 to 4 carbon atoms, an alkoxy group having 1 to 4 carbon atoms, a nitro group, a carboxy group, a hydroxy group or an amino group; a hydroxycarboxylic acid compound represented by the following general formula (V):

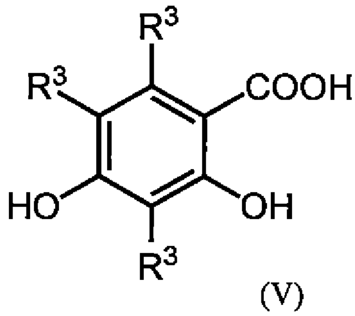

(V)

wherein in the formula, $R^3$s are each independently a hydrogen atom, an alkyl group having 1 to 4 carbon atoms, an alkenyl group having 2 to 4 carbon atoms, an alkynyl group having 2 to 4 carbon atoms or an alkoxy group having 1 to 4 carbon atoms; an organic compound represented by the following general formulas (VI) to (VIII):

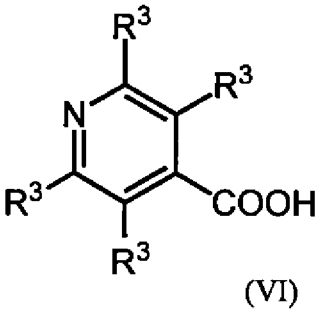

(VI)

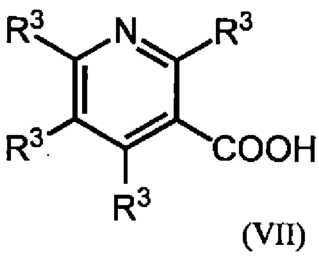

(VII)

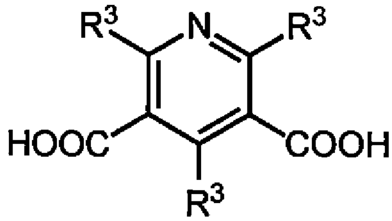

(VIII)

wherein in the formulas, $R^3$s are each independently a hydrogen atom, an alkyl group having 1 to 4 carbon atoms, an alkenyl group having 2 to 4 carbon atoms, an alkynyl group having 2 to 4 carbon atoms or an alkoxy group having 1 to 4 carbon atoms; and an organic compound represented by the following general formulas (IX) to (XII):

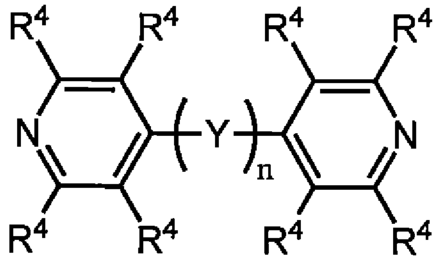

(IX)

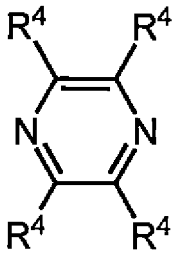

(X)

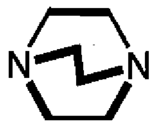

(XI)

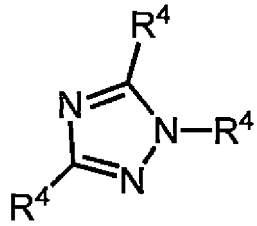

(XII)

wherein in the formulas, Ys are each independently an oxygen atom, a sulfur atom, $-CH_2-$ , -CH(OH)-, -CO-, -NH-, $-C_2N_4-$, -C=C-, $-C_2H_2-$ or $-C_6H_4-$, and $R^4$s are each independently a hydrogen atom, a halogen atom, an alkyl group having 1 to 4 carbon atoms, an alkoxy group having 1 to 4 carbon atoms, a formyl group, an acyloxy group having 1 to 4 carbon atoms, an alkoxycarbonyl group having an alkoxy group having 1 to 4 carbon atoms, a nitro group, a cyano group, a carboxy group, an amino group, a monoalkylamino group having 1 to 4 carbon atoms, a dialkylamino group having alkyl groups having 1 to 4 carbon atoms, or an acylamino group having 1 to 4 carbon atoms, and n is an integer from 0 to 3.

FIG. 1

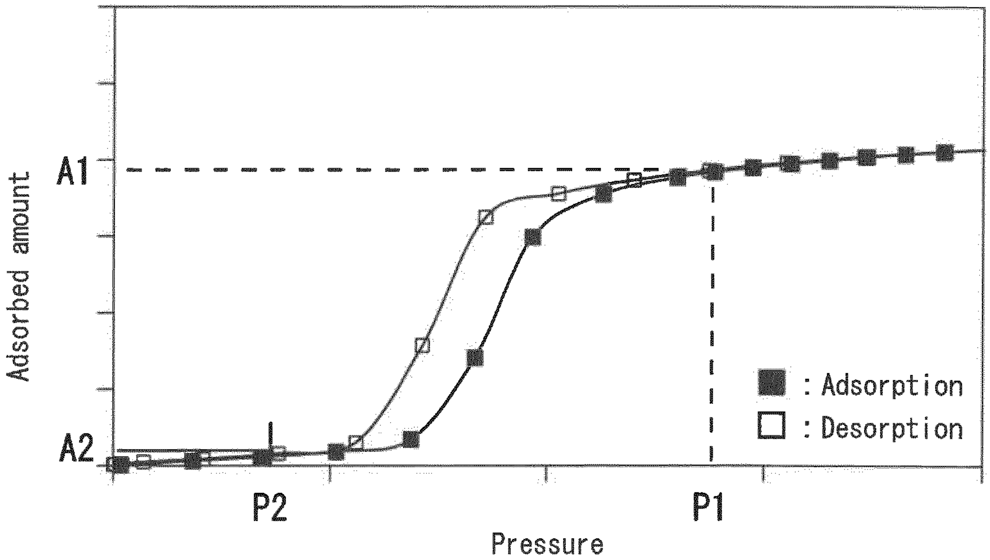
A1
A2
Adsorbed amount
P2
P1
Pressure
: Adsorption
: Desorption

FIG. 2

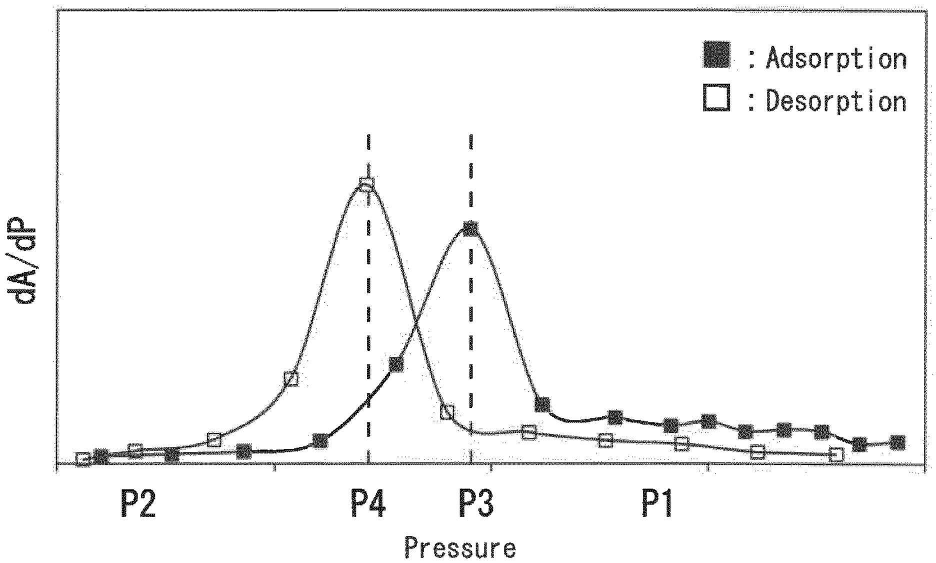
: Adsorption
: Desorption
dA/dP
P2
P4
P3
P1
Pressure

FIG. 3

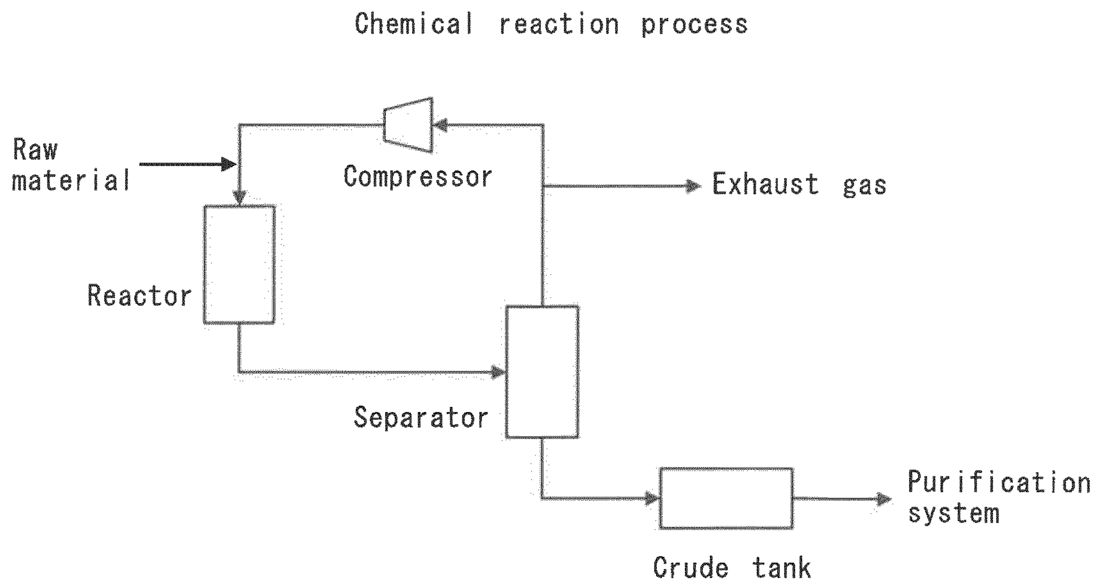
Chemical reaction process
Raw material
Compressor
Exhaust gas
Reactor
Separator
Purification system
Crude tank

FIG. 4

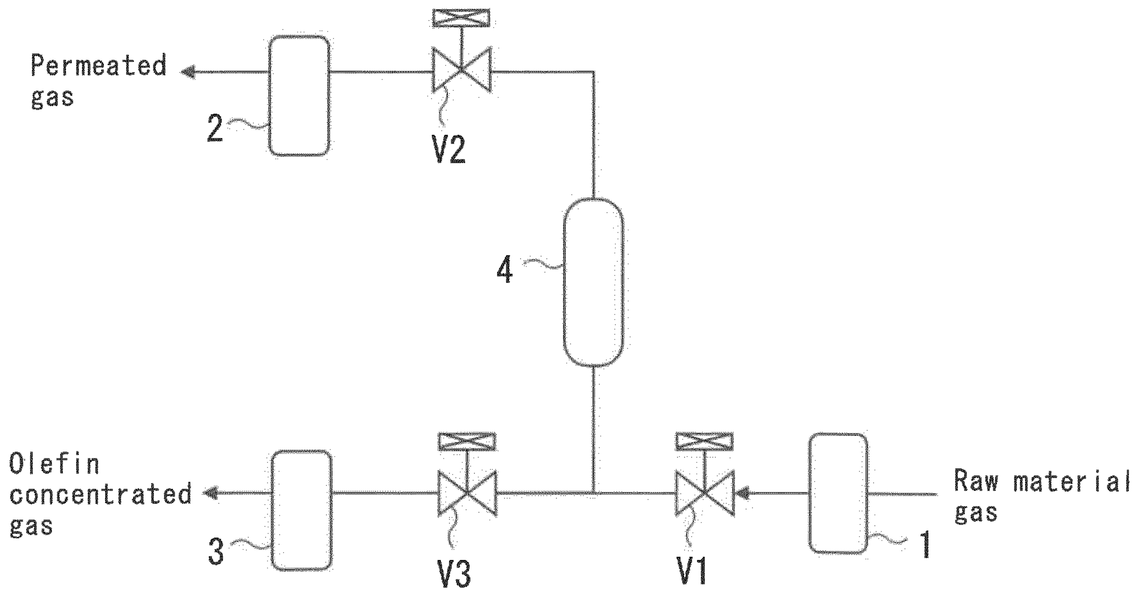
Permeated gas
2
V2
4
Olefin concentrated gas
3
V3
V1
Raw material gas
1

FIG. 5

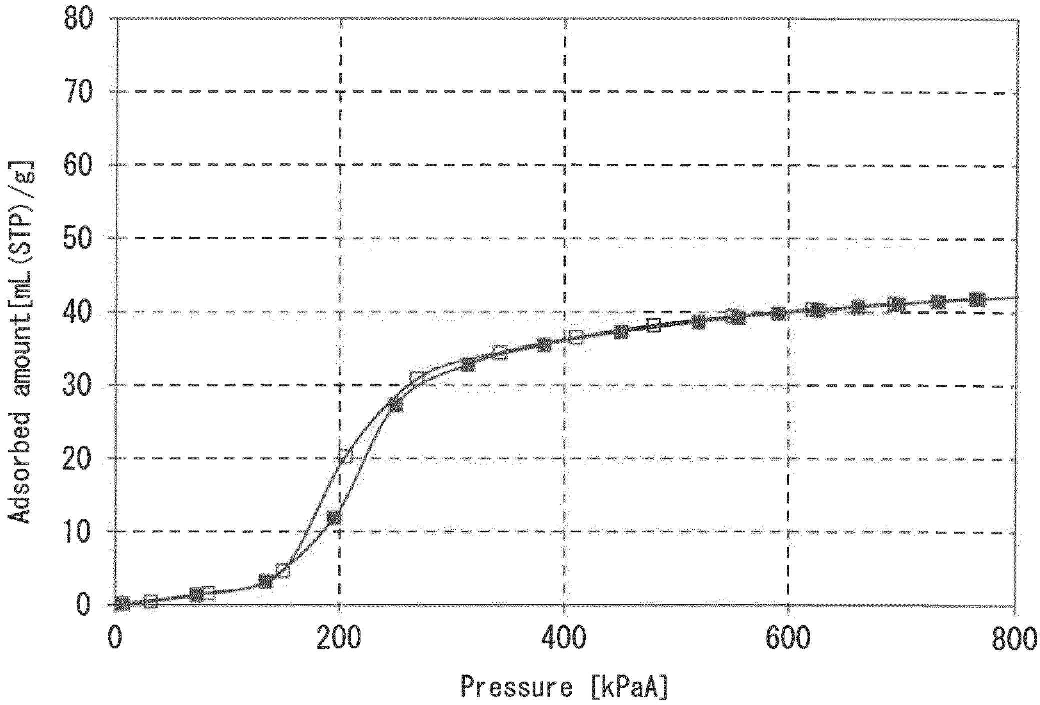
80
70
60
50
40
30
20
10
0
Adsorbed amount[mL(STP)/g]
0
200
400
600
800
Pressure [kPaA]

FIG. 6

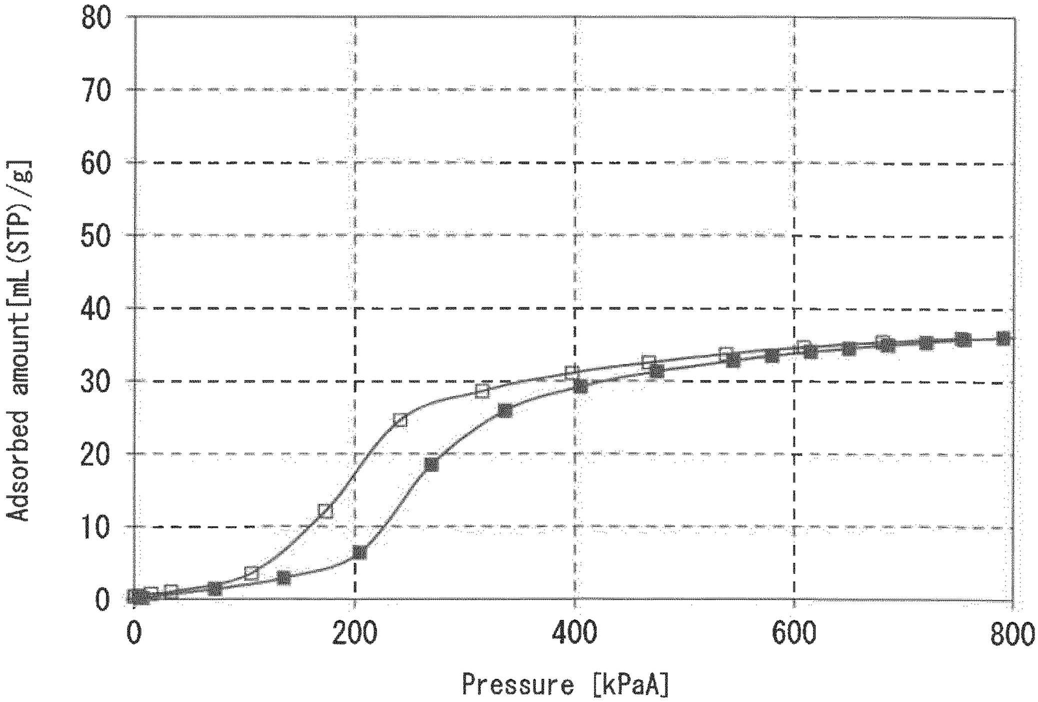
80
70
60
50
40
30
20
10
0
Adsorbed amount[mL(STP)/g]
0
200
400
600
800
Pressure [kPaA]

FIG. 7

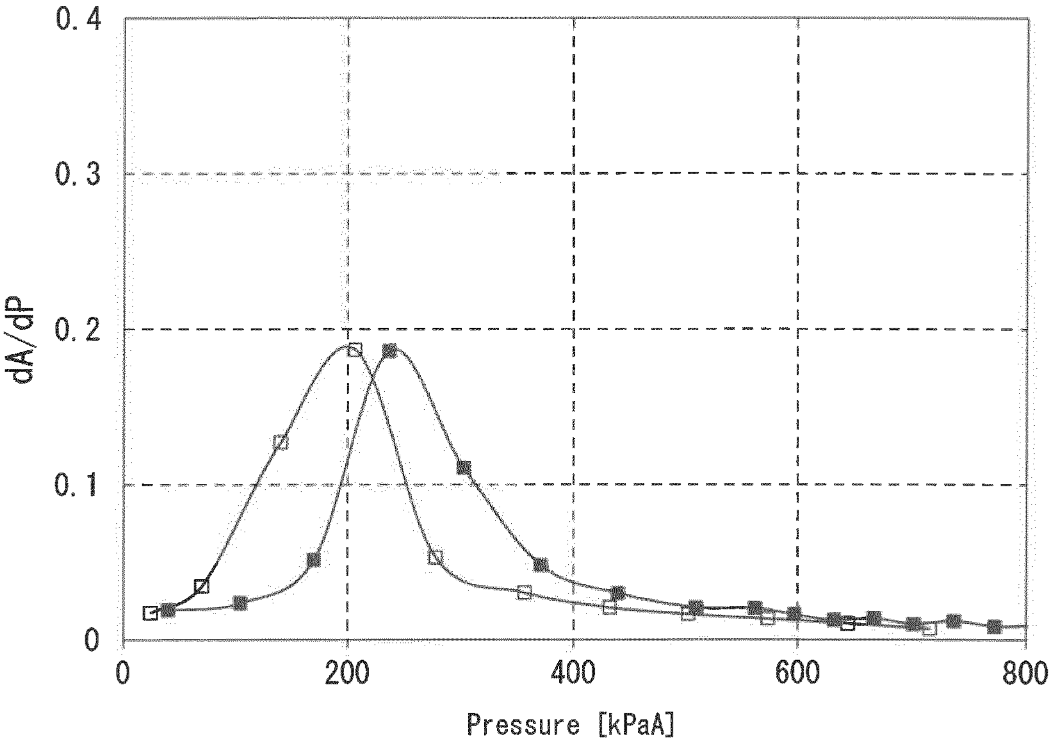
0.4
0.3
0.2
0.1
0
dA/dP
0
200
400
600
800
Pressure [kPaA]

FIG. 8

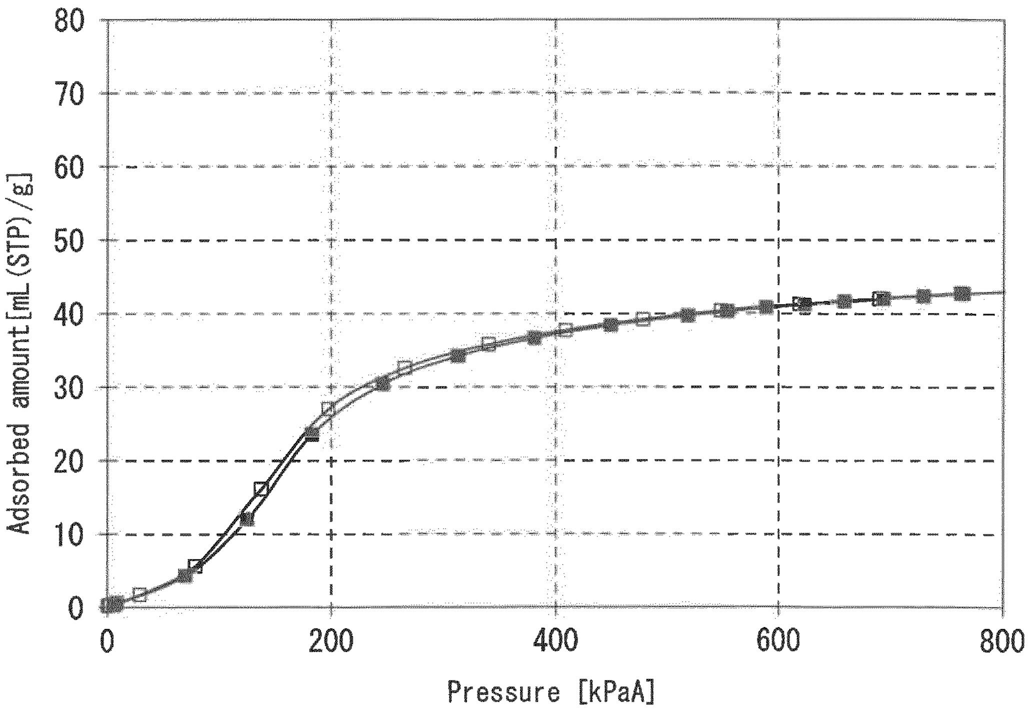
80
70
60
50
40
30
20
10
0
Adsorbed amount [mL (STP)/g]
0
200
400
600
800
Pressure [kPaA]

FIG. 9

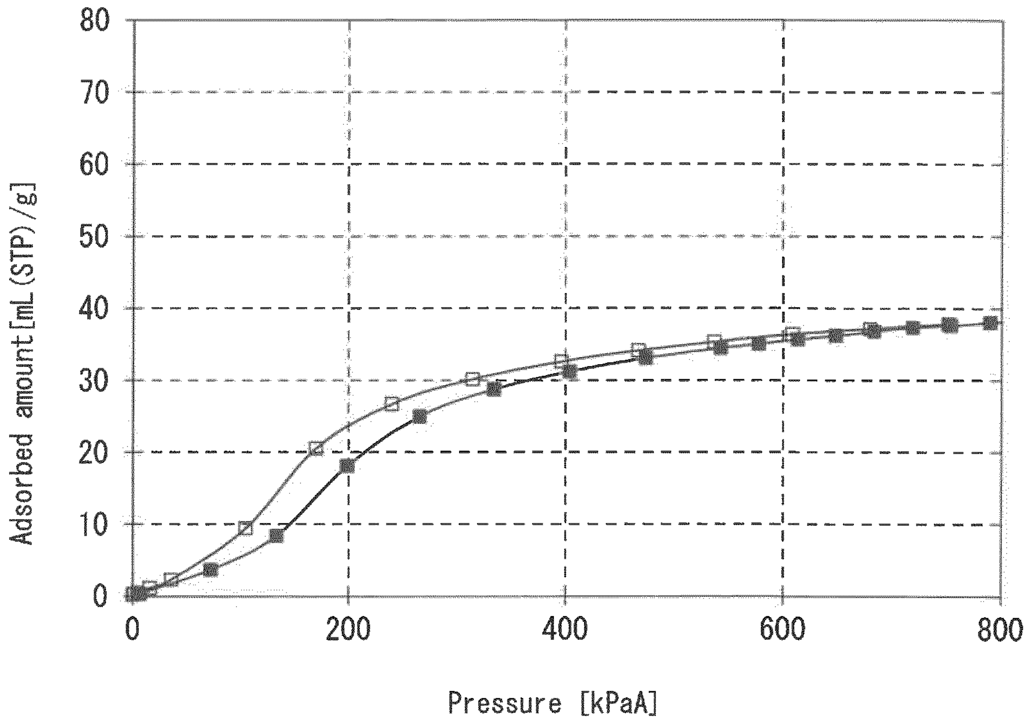
80
70
60
50
40
30
20
10
0
Adsorbed amount [mL (STP) /g]
0
200
400
600
800
Pressure [kPaA]

FIG. 10

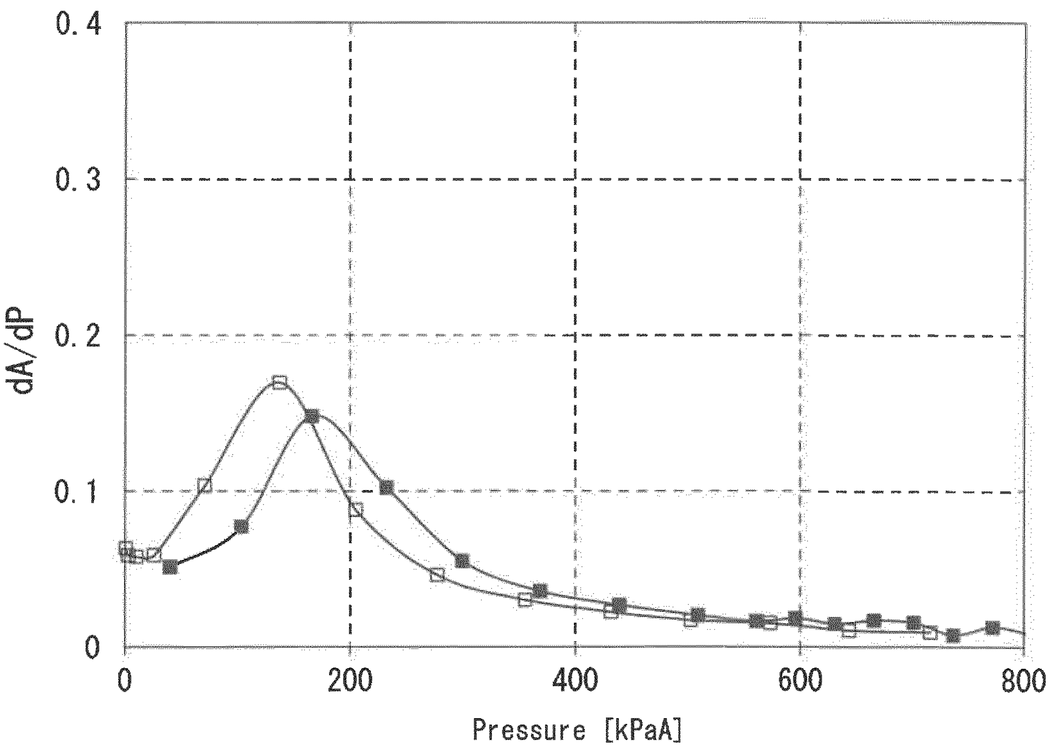
0.4
0.3
0.2
0.1
0
dA/dP
0
200
400
600
800
Pressure [kPaA]

FIG. 11

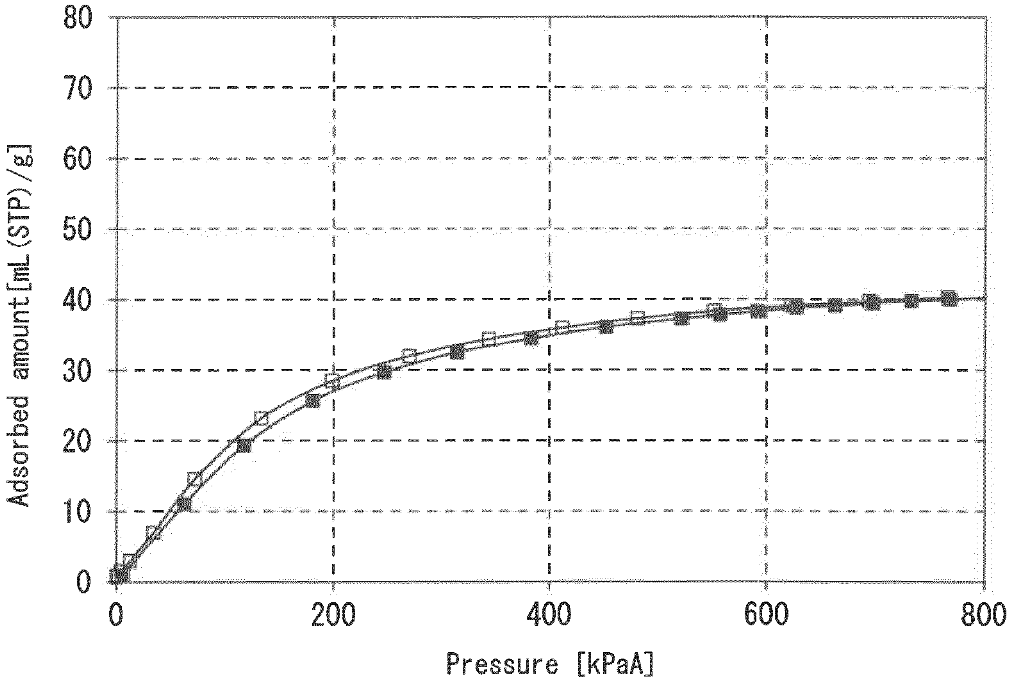
80
70
60
50
40
30
20
10
0
0
200
400
600
800
Adsorbed amount[mL(STP)/g]
Pressure [kPaA]

FIG. 12

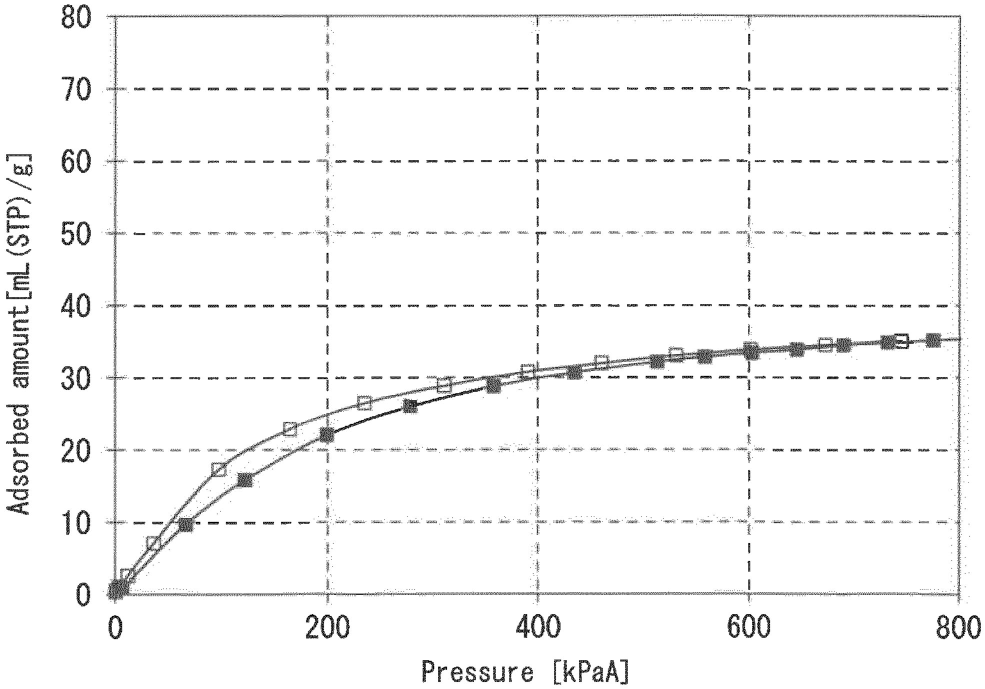
80
70
60
50
40
30
20
10
0
0
200
400
600
800
Adsorbed amount[mL(STP)/g]
Pressure [kPaA]

FIG. 13

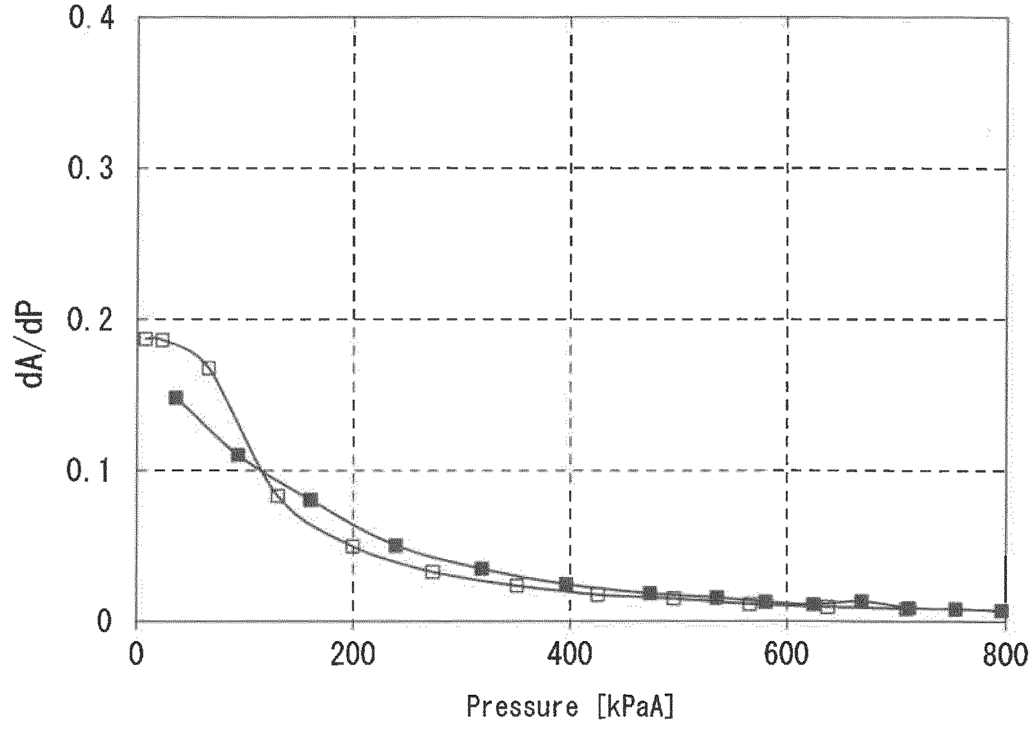
0.4
0.3
0.2
0.1
0
dA/dP
0
200
400
600
800
Pressure [kPaA]

FIG. 14

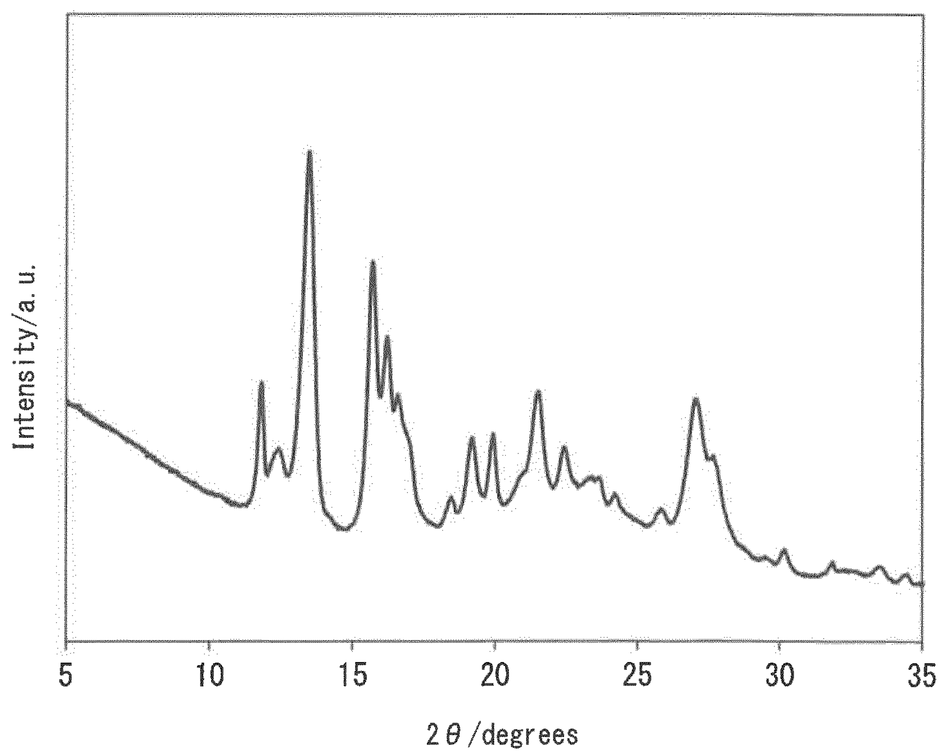
Intensity/a.u.
5
10
15
20
25
30
35
2θ/degrees

FIG. 15

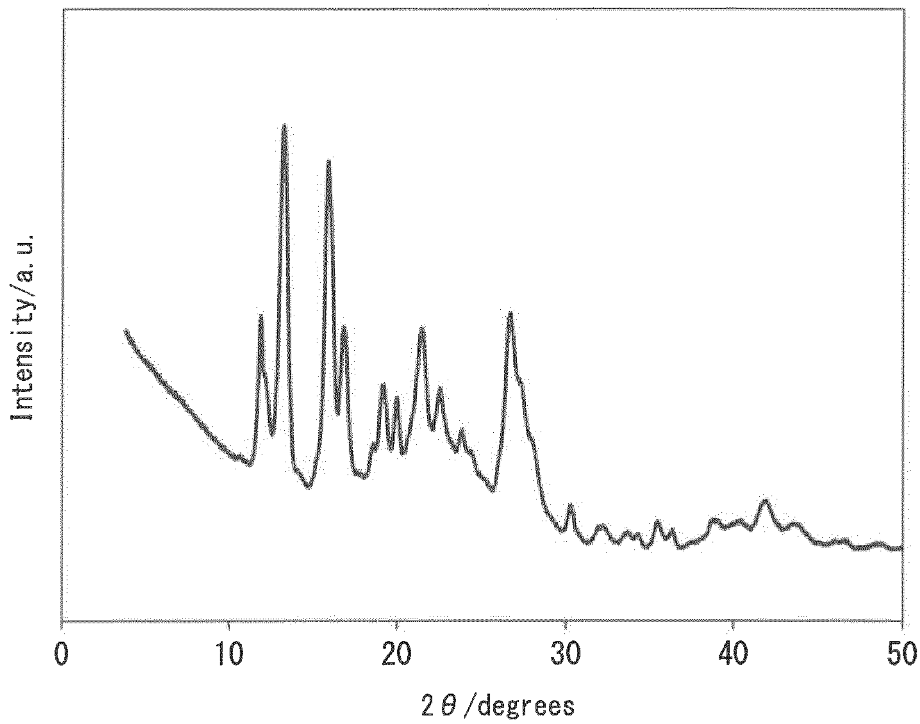
Intensity/a.u.
0
10
20
30
40
50
2θ/degrees

FIG. 16

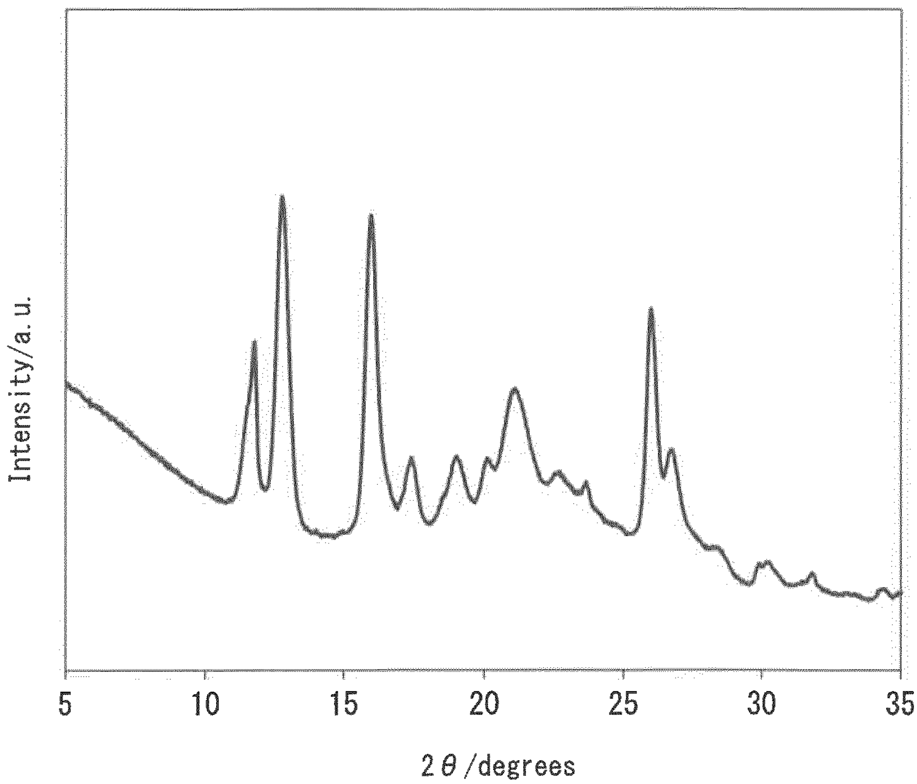
Intensity/a.u.
5
10
15
20
25
30
35
2θ/degrees

## INTERNATIONAL SEARCH REPORT

International application No. PCT/JP2020/029335

**A. CLASSIFICATION OF SUBJECT MATTER**

Int.Cl. C07C7/12(2006.01)i, C07C11/04(2006.01)i, B01D53/047(2006.01)i, B01J20/22(2006.01)i, B01J20/34(2006.01)i
FI: C07C7/12, B01D53/047, B01J20/22A, B01J20/34E, C07C11/04

According to International Patent Classification (IPC) or to both national classification and IPC

**B. FIELDS SEARCHED**

Minimum documentation searched (classification system followed by classification symbols)
Int.Cl. C07C7/12, C07C11/04, B01D53/047, B01J20/22, B01J20/34, C08F2/00-2/60

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | WO 2013/115033 A1 (TOYOBO CO., LTD.) 08.08.2013 (2013-08-08), claims 1-5, paragraphs [0044], [0074], [0101]-[0106], fig. 3, 4 | 1-8 |
| Y | WO 2014/103778 A1 (SHOWA DENKO KK) 03.07.2014 (2014-07-03), claims 1-12, paragraphs [0085]-[0091], fig. 3-5 | 1-8 |
| Y | JP 2013-63951 A (SUMITOMO CHEMICAL CO., LTD.) 11.04.2013 (2013-04-11), claims 1-17, paragraphs [0078]-[0087], fig. 14-25 | 1, 2, 4, 6-8 |
| Y | US 2003/0073788 A1 (GOLDEN, T. C.) 17.04.2003 (2003-04-17), claim 1 | 1-8 |

☒ Further documents are listed in the continuation of Box C. ☒ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| 08.09.2020 | 24.09.2020 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT

International application No. PCT/JP2020/029335

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2003-144827 A (AIR PRODUCTS & CHEMICALS INC.) 20.05.2003 (2003-05-20), claims 1, 8, 22, paragraphs [0002], [0003], [0008], [0035]-[0040] | 1-8 |
| Y | JP 2018-523730 A (EXXONMOBIL CHEMICAL PATENTS INC.) 23.08.2018 (2018-08-23), paragraph [0011] | 1-8 |
| Y | US 4769047 A (SHELL OIL COMPANY) 06.09.1988 (1988-09-06), claims 1-5 | 1-5, 7, 8 |
| Y | JP 2011-219471 A (SUMITOMO CHEMICAL CO., LTD.) 04.11.2011 (2011-11-04), claim 1, paragraph [0016] | 1, 2, 4, 7, 8 |
| A | WO 2015/012067 A1 (SHOWA DENKO KK) 29.01.2015 (2015-01-29), entire text | 1-8 |
| A | WO 2015/012069 A1 (SHOWA DENKO KK) 29.01.2015 (2015-01-29), entire text | 1-8 |
| A | JP 2018-089577 A (L'AIR LIQUIDE SOCIETE ANONYME POUR L'ETUDE ET L'EXPLOITATION DES PROCEDES GEORGES CLAUDE) 14.06.2018 (2018-06-14), entire text | 1-8 |
| A | WO 2015/012396 A1 (HOKKAIDO UNIVERSITY) 29.01.2015 (2015-01-29), entire text | 1-8 |

Form PCT/ISA/210 (second sheet) (January 2015)

**INTERNATIONAL SEARCH REPORT**
Information on patent family members

International application No.
PCT/JP2020/029335

| | | |
|---|---|---|
| WO 2013/115033 A1 | 08.08.2013 | (Family: none) |
| WO 2014/103778 A1 | 03.07.2014 | US 2015/0329563 A1<br>claims 1-12, paragraphs [0115]-[0121], fig. 3-5<br>KR 10-2015-0032584 A<br>CN 104768905 A |
| JP 2013-63951 A | 11.04.2013 | (Family: none) |
| US 2003/0073788 A1 | 17.04.2003 | EP 1302478 A1<br>claim 1 |
| JP 2003-144827 A | 20.05.2003 | US 2003/0070546 A1<br>claims 1, 8, 22,<br>paragraphs [0002], [0003],<br>[0014]-[0019], [0072]-[0077]<br>EP 1302233 A2 |
| JP 2018-523730 A | 23.08.2018 | WO 2017/023433 A1<br>paragraph [0018]<br>CN 107849168 A<br>KR 10-2018-0022937 A<br>US 2018/0162962 A1 |
| US 4769047 A | 06.09.1988 | GB 2206354 A<br>claims 1-5 |
| JP 2011-219471 A | 04.11.2011 | WO 2011/118823 A1<br>claim 1, paragraph [0016] |
| WO 2015/012067 A1 | 29.01.2015 | US 2016/0159823 A1<br>entire text<br>KR 10-2015-0132579 A<br>CN 107805260 A |
| WO 2015/012069 A1 | 29.01.2015 | US 2016/0159712 A1<br>entire text |
| JP 2018-089577 A | 14.06.2018 | US 2018/0155258 A1<br>entire text |
| WO 2015/012396 A1 | 29.01.2015 | (Family: none) |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2009208028 A
- JP 4994398 B
- JP 4834048 B


### Non-patent literature cited in the description


- **KAZUHIRO UEMURA ; SUSUMU KITAGAWA.** *Expected materials for the future,* 2002, vol. 2, 44-51
- **YASUSHI TAKEUCHI.** Adsorption Separation. *Baifukan,* 2000, 35